# EUROPEAN PATENT SPECIFICATION

(11) **EP 4 499 783 B1**
(45) Date of publication and mention of the grant of the patent: **17.12.2025**
(21) Application number: 23715084.2
(22) Date of filing: 24.03.2023
(51) Int. Cl.: C10G 2/00, C10G 69/12, C07C 29/153, C07C 29/151, C10G 50/00, C10G 3/00

(54) **PROCESS AND PLANT FOR PRODUCING HYDROCARBONS FROM A GAS COMPRISING CO2**
VERFAHREN UND ANLAGE ZUR HERSTELLUNG VON KOHLENWASSERSTOFFEN AUS EINEM CO2-HALTIGEN GAS
PROCÉDÉ ET INSTALLATION POUR LA PRODUCTION D'HYDROCARBURES À PARTIR D'UN GAZ COMPRENANT DU CO2

(30) Priority: 29.03.2022 EP 22165008
(43) Date of publication of application: 05.02.2025
(73) Proprietor: Topsoe A/S, 2800 Kgs. Lyngby (DK)
(72) Inventor: JOENSEN, Finn, 2970 Hørsholm (DK); SCHJØDT, Niels Christian, 4330 Hvalsø (DK); BEATO, Pablo, 2150 Nordhavn (DK); BROGAARD, Rasmus Yding, 2800 Kgs. Lyngby (DK); SOMMER, Linn Edda, 2100 Copenhagen Ø (DK)
(74) Representative: Topsoe A/S
(86) International application number: PCT/EP2023/057656
(87) International publication number: WO 2023/186736

(56) References cited:
- CN-A- 108 970 638
- CN-A- 113 289 677
- US-A1- 2015 191 666
- US-A1- 2020 399 190

## Description

The present invention relates to the production of jet fuel from CO₂ by combining CO₂-to-methanol (CO₂-to-MeOH) and methanol-to-olefin (MTO) syntheses in a single step for producing olefins suitable for oligomerization and hydrogenation to hydrocarbons boiling in the jet fuel range, thus usable as jet fuel, particularly as sustainable aviation fuel (SAF), optionally also hydrocarbons boiling in the diesel range. More generally, the present invention relates to the production of jet fuel from a synthesis gas, such as a gas comprising CO₂, by combining the CO₂-to-methanol (CO₂-to-MeOH) and methanol-to-olefin (MTO) syntheses in a single step.

Currently, it is known to produce methanol from CO₂ by the so-called direct CO₂-hydrogenation process. The hydrogenation of CO₂ to produce methanol suffers from unfavorable thermodynamics and requires a high pressure, typically about 70 bar, whereas the conversion of oxygenates such as methanol and/or dimethyl ether to olefins may be conducted at low pressure, for instance in the range 1-30 bar.

It is therefore known to combine the two process steps into one that breaks the thermodynamic constraints and enables the combined synthesis to olefins to take place at moderate pressure (20-60 bar), yet typically, existing processes produce mainly light (C2-C4) olefins.

For instance, WO2018045652A1 discloses catalyst systems such as e.g. Zn/Zr oxides with SAPO-34 or ZSM-5, Pd/ZnO-SAPO-34, Pd/ZnZr oxides-SAPO-34. The catalysts are applied for (one-step) conversion of CO₂ to olefins.

CN111111765A discloses various catalyst systems with metal oxide combinations containing Zn and/or In, and another metal component selected from a shorter listing including Zr, Ce amongst others and zeotypes such as SAPO-18, SAPO-34, AIPO-18, or AIPO-34.

CN111558393A discloses a catalyst comprising zeolite RUB-13 and a composite metal oxide with metals from group IIB and group IVB and from the lanthanides. In the given examples, the hydrogenation of carbon dioxide to propylene and butene over Zn-Ce-Zr / RUB-13 is compared to the respective materials with ZSM-5 and SAPO-34.

WO2020060591A1 discloses a process for production of C2-C5 hydrocarbons using a bifunctional catalyst with a metal oxide component and a microporous catalyst component. To a CO+H₂ feed, a stream with CO₂ and H₂O is added. The microporous component of the catalyst can be a molecular sieve with eight (8) membered rings (8-MR) such as SAPO-34 and the metal oxidic component can comprise zinc.

CN 113289677 A discloses a dual catalyst for the conversion of s syngas to aromatic hydrocarbons, the dual catalyst comprising ZSM-48 and a mixture of cerium oxide-zirconia and zinc-oxide-zirconia. This citation is not related to the production of jet fuel. This citation is thus at least silent on oligomerization and hydrogenation to produce jet fuel.

CN 108970638 A discloses a process for direct conversion in a single reaction of syngas to gasoline. This citation is not related to the production of jet fuel. This citation is thus at least silent on oligomerization and hydrogenation to produce jet fuel.

US 2020399190 A1 discloses the conversion of syngas to distillates via conversion to methanol, then to olefins and subsequently oligomerization of the olefins. This citation is at least silent on the provision of a dual catalyst which is free of copper.

US 2015191666 A1 discloses a process for producing jet fuel by syngas conversion to methanol and dimethyl ether (DME), conversion of these to olefins, oligomerization of olefins and hydrotreating to produce a jet fuel cut. This citation is at least silent on providing a dual catalyst which is free of copper.

It would be desirable to provide a superior process for the conversion of CO₂ into C3-C8 olefins, in particular C4-C8 olefins which are particularly suitable as intermediates for making hydrocarbons in the transportation fuel range, i.e. by having the constituents of diesel and jet fuel C8 to C20+ paraffins, in particular jet fuel (C8-C16) as sustainable aviation fuel (SAF).

Due to society concerns about global climate change and the resulting political pressure on the aviation industries, the market for SAFs is expected to increase substantially during the next decades. A pre-condition for the use of any SAF as aviation turbine fuel is an ASTM certification. So far, only a small number of processes, producing SAF or synthetic paraffinic kerosene (SPK) fuels have been approved by ASTM International (ASTM) Method D7566 for blending into jet fuel at levels up to 50%. One important general requirement is therefore that the synthetic part of SAF (50 vol%) must be virtually free from aromatics, while the final SAF blend can contain up to 26.5 vol% aromatics.

Furthermore, in the chemical industry, the normal practice is to produce propene (propylene) and jet fuel in separate processes. While the MTO reaction may be used to produce propylene along with other hydrocarbons, purification of the propylene to an acceptable grade is normally perceived as being costly and energy intensive. Synthetic jet fuel, in particular SAF, is not produced industrially yet on a large scale and one reason may be related to the difficulties in obtaining sufficient economy in such process.

It would therefore also be desirable to provide a simple process for the conversion of CO₂ into the valuable products propylene and sustainable aviation fuel (SAF); more specifically, to be able in a simple manner to produce from CO₂, not only a valuable fuel such as SAF, but also a valuable chemical, particularly propylene, more specifically chemical grade or polymer-grade propylene.

As used herein, "C-MTO" (carbon to methanol to olefins) means the conversion of carbon oxides to olefins, in particular the conversion of carbon oxides incl. CO₂ under the presence of H₂ to an oxygenate such as methanol, and the conversion of the oxygenate to olefins.

As used herein, "synthesis gas" is used interchangeably with "syngas" and means a gas mixture comprising a carbon oxide (CO and/or CO₂) and H₂. For instance, the synthesis gas comprises H₂ and the carbon oxide(s) in a molar ratio of at least 3:1. The term "CO and/or CO₂" means any of: CO, CO₂, or a combination thereof. Hence, the syngas is for instance a mixture of CO₂ and H₂; this syngas may also be referred to as a "gas comprising CO₂".

As used herein, "OLI" means oligomerization.

As used herein, "HYDRO" means hydrogenation.

As used herein, the term "hydroprocessing" means any of hydrotreating, hydrocracking, hydrogenation (HYDRO), or combinations thereof

As used herein, "HYDRO/OLI" means a single combined step comprising hydrogenation and oligomerization. The term is used interchangeably with "OLI/HYDRO". It would be understood that OLI represents normally the first step and HYDRO the second step. As used herein, "C-MTJP" means carbon to methanol to jet fuel and propylene and is interchangeable with the term "overall process" or "overall process and plant", which means a process/plant combining C-MTO, OLI and HYDRO, whereby syngas is converted into propylene and jet fuel. The overall process and plant may also include a front-end section for producing the syngas.

As used herein, the terms "jet fuel" and "hydrocarbons boiling in the jet fuel range" are used interchangeably and have the meaning of a mixture of C8-C16 hydrocarbons boiling in the range of about 130-300°C at atmospheric pressure. As used herein, "diesel" and "hydrocarbons boiling in the diesel fuel range" are used interchangeably and have the meaning of a mixture of C20+ hydrocarbons boiling in the range 120-360°C, for instance 160-360°C.

As used herein, "SAF" means sustainable aviation fuel or aviation turbine fuel, in compliance with ASTM D7566 and ASTM D4054.

As used herein, the terms "methanol" and "dimethyl ether" are used interchangeably with the terms MeOH and DME, respectively. "MeOH/DME" means MeOH and/or DME. The term "MeOH and/or DME" means any of: MeOH, DME or combinations thereof. Same interpretation applies when utilizing "and/or" throughout this application. For instance, the term "CO and/or CO₂" means any of: CO, CO₂, or a combination (mixture) thereof. For instance, the term "ethane/ethylene" means "ethane and/or ethylene", i.e. any of: ethane, ethylene, or a combination thereof.

As used herein, the term "olefin product stream" means a stream exiting C-MTO (thus exiting a C-MTO reactor) and also means a hydrocarbon stream rich in olefins comprising higher and lower olefins, and optionally also aromatics, paraffins, iso-paraffins and naphthenes, and in which the combined content of higher and lower olefins is at least 25 wt%, such as 30 wt% or 50 wt%. For example, the olefin product stream comprises C2 olefins (C2=) and C3-C8 olefins (C3=-C8=). The olefin product stream may also be an olefin product stream from which C3 olefins (C3=) have been removed.

As used herein, the term "higher olefins" means olefins having three (3) or more carbons (C3+ olefins), in particular C3-C8 olefins (C3= - C8=), including olefins having four (4) or more carbons (C4+ olefins), in particular C4-C8 olefins (C4= - C8=).

As used herein, the term "lower olefins" means an olefin having two carbons, i.e. ethylene (C2-olefin or synonymously C2= or ethene).

As used herein, the terms C3 olefin, C3=, propene, propylene, are used interchangeably.

As used herein, the term "C3 olefin product stream" means a stream rich in propylene, suitably at least 93 vol% propylene corresponding to chemical grade propylene, or for instance at least 99.5 vol% corresponding to polymer grade propylene.

As used herein, the term "high content of higher olefins" means that the weight ratio in the olefin product stream of higher olefins to lower olefins is above 1, suitably above 10, for instance 20-90 such as 70-80.

As used herein, the term "selectivity to higher olefins" means the weight ratio of higher to lower olefins i.e. weight ratio of higher olefins to ethylene. "High selectivity to higher olefins" or "higher selectivity to higher olefins" means a weight ratio of higher to ethylene of above 10.

As used herein, the terms "C2-light fraction" means containing C2= and C1-2 hydrocarbons, and optionally also CO, CO₂ and H₂.

As used herein, the term "lower hydrocarbons" means C1-2 (e.g. methane, ethane) and optionally also C2=.The term is also used interchangeably with the term "light paraffins".

As used herein, the term "essentially free or ethylene" or "free of ethylene" or "free of propylene" means 1 wt% or lower.

As used herein, the term "essentially free of aromatics", "substantially free of aromatics", "aromatic-free" or "low aromatics" means less than 5 wt%, e.g. 1 wt% or even less than 1 wt%. Aromatics include benzene (B), toluene (T), xylene (X) and ethylbenzene. It would be understood that the "xylene" is any one of three isomers of dimethylbenzene, or a combination thereof.

As used herein, the terms "catalyst comprising a zeolite" and "zeolite catalyst" are used interchangeably.

As used herein, the term "silica to alumina ratio (SAR)" means the mole ratio of SiO₂ to Al₂O₃.

As used herein, the term "significant amount of paraffins" means 5-20 wt%, such as 10-15 wt% in the olefin product stream.

As used herein, the term "comprising" or "comprises" may also have the meaning of "comprising only", i.e. "consisting of", or "comprises only", i.e. "consists of", respectively.

As used herein, the term "suitably" means "optionally", i.e. an optional embodiment. As used herein, the term "present invention" or simply "invention" may be used interchangeably with the term "present application ", or simply "application".

Other definitions are provided in connection with one or more embodiments of the invention.

Accordingly, the present invention is a process for producing a hydrocarbon stream comprising hydrocarbons boiling in the jet fuel range, said process comprising:
a) passing a synthesis gas (syngas) stream comprising carbon dioxide and hydrogen over a fixed bed in a conversion reactor, the fixed bed comprising a dual-function catalyst active in the conversion of syngas to the oxygenate(s) methanol (MeOH) and/or dimethyl ether (DME), and the conversion of said oxygenate(s) to an olefin product stream; wherein the dual-function catalyst comprises a MeOH synthesis catalyst in which the MeOH synthesis catalyst is a Cu and Cr-free catalyst comprising an oxide of Zn in combination with an oxide of any of Zr, Al, Si, Ti, Ce, La, Ga, In, Mo, Mn, Mg, Y, or combinations thereof; and an oxygenate conversion catalyst comprising a zeolite with a framework having a 10-ring pore structure, in which the 10-ring pore structure comprises a unidimensional (1-D) pore structure selected from any of: *MRE (ZSM-48), MTT (ZSM-23), TON (ZSM-22), or combinations thereof;
b) passing at least a portion of the olefin product stream through an oligomerization step over an oligomerization catalyst, and optionally subsequently conducting a separation step, for thereby producing an oligomerized stream;
c) passing at least a portion of the oligomerized stream through a hydrogenation step over a hydrogenation catalyst, and optionally subsequently conducting a separation step, for thereby producing said hydrocarbon stream comprising hydrocarbons boiling in the jet fuel range, optionally also comprising hydrocarbons boiling in the diesel fuel range.

Accordingly, in an embodiment, step b) further comprises subsequently conducting a separation step, for thereby producing said oligomerized stream; and/or step c) further comprises subsequently conducting a separation step, for thereby producing said hydrocarbon stream comprising hydrocarbons boiling in the jet fuel range, optionally also comprising hydrocarbons boiling in the diesel fuel range.

It would be understood, that C-MTO corresponds to step a); OLI corresponds to step b); and HYDRO corresponds to step c).

The MeOH synthesis catalyst in step a) has a low hydrogenation activity towards production of paraffins, i.e. it avoids olefins becoming hydrogenated to paraffins, while at the same time providing significant catalytic activity for the conversion of methanol. This contrasts typical catalysts for methanol conversion of a synthesis gas, such as Cu-based catalysts, e.g. Cu/Al₂O₃, which have a high hydrogenation activity towards production of paraffins.

The syngas may be a mixture of CO₂ and H₂, or a mixture of CO, CO₂ and H₂. As is well-known in the art, for methanol synthesis the syngas requires a module M=(H₂-CO₂)((CO+CO₂) in the range 1.90-2.20. It would be understood that the concentrations in the formula are by vol. basis. Where the syngas comprises CO, the CO is suitably in step a) first converted to CO₂ and the latter then to methanol.

It would also be understood that the zeolite term *MRE may be used interchangeably with the term ZSM-48; MTT may be used interchangeably with the term ZSM-23; and TON may be used interchangeably with the term ZSM-22.

Suitably, the olefin product stream in step a) comprises C3 olefins (C3=) and C3-C8 olefins (C3=-C8=), such as C4-C8 olefins.

The present invention combines thereby a methanol synthesis catalyst having a low hydrogenation activity towards production of paraffins, with an oxygenate conversion catalyst characterized by being a uni-dimensional (1-D) 10-membered ring zeolite, preferably ZSM-48 or, alternatively, ZSM-22 and/or ZSM-23. This combination provides for a facile route to produce the intermediate-size (C4-C8) olefins and even higher sized olefins such as C9-C11 olefins, or even higher, which are suitable as feed for subsequent oligomerization and hydrogenation, thus producing the long-chain (C8-C20+) paraffins and (predominantly) isoparaffins which are valuable components in jet fuel and diesel.

It would be understood that the term "dual-function" catalyst means a catalyst which is active in the conversion of syngas to the oxygenate(s) methanol (MeOH) and/or dimethyl ether (DME), and the conversion of said oxygenate(s) to an olefin product stream;

Hence, while it is known to use a catalyst comprising zeolites such as ZSM-5 or SAPO for (one-step) conversion of CO₂ to olefins, and it is also known to use a catalyst comprising ZSM-48 for converting MeOH into olefins, the present invention lies specifically in the combination of CO₂-to-MeOH-to-olefins in a conversion reactor and thereafter to jet fuel, by passing syngas and thereby CO₂ through the dual-function catalyst comprising the MeOH-catalyst having low hydrogenation activity towards production of paraffins, i.e. a Cu and Cr-free catalyst comprising at least an oxide of any of Zn and Zr, or as further defined in appended claim 1, and the 10-ring 1-D zeolite (ZSM-48, ZSM-22, ZSM-23).

There is a surprising synergistic effect associated thereto:
It has namely been found, that high MeOH partial pressure (P_{MeOH}) tends to increase selectivity to aromatics (and light paraffins: C2-C5 paraffins), thereby reducing the selectivity to the desired olefins, C3-C8 or C4-C8 olefins. The higher P_{MeOH}, the higher the selectivity to aromatics (and light paraffins) irrespective of the temperature. The invention enables the continuous removal and thus further reaction of MeOH being produced in the fixed bed of a conversion reactor so that the MeOH-concentration decreases and is kept low through the C-MTO reactor. Thereby, by continuously removing MeOH by converting it into hydrocarbons, predominantly olefins, the production of MeOH is thermodynamically favored, while at the same time increasing the selectivity to the desired olefins for jet fuel (C3-C8, or C4-C8), as i.a. the content of aromatics and thereby lighter paraffins (C3-C5) and which are difficult to upgrade to jet fuel, is kept low. Hence, the invention enables maintaining the P_{MeOH} as low as possible throughout the fixed bed in order to maximize the selectivity to olefins, by applying the dual-function catalyst, capable of simultaneously converting the synthesis gas into MeOH and MeOH into olefins. Furthermore, by the MeOH synthesis catalyst having low hydrogenation activity towards production of paraffins, the invention enables also to keep the aromatics content low and thus increase the selectivity to the desired olefins. The desired olefins may also comprise olefins after step b) already in the jet fuel range (C8-C16).

In an embodiment, in step a), in the MeOH synthesis catalyst at least 80 wt%, e.g. at least 90 wt%, are oxides of Zn in combination with oxides of Zr and/or Al; and optionally, up to 20 wt% e.g. up to 10 wt% are oxides of any of: Si, Ti, Ce, La, Ga, In, Mo, Mn, Mg, Y.

For instance, the MeOH synthesis catalyst is selected from any of: oxides of Zn-Zr, ZnO/ZrO₂, ZnO/(CeO₂/ZrO₂), or combinations thereof.

For instance, the oxides of Zn-Zr, are prepared by co-precipitation.

For instance, the ZnO/ZrO₂ MeOH catalyst is a catalyst comprising ZnO supported on a ZrO₂ carrier. It may also comprise additional components such as SiO₂ and/or Al₂O₃.

For instance, the MeOH synthesis catalyst comprises oxides of Zn and Al such as ZnO, Al₂O₃ and ZnAl₂O₄. Suitably, the MeOH synthesis catalyst is ZnAl₂O₄ i.e. zinc aluminium spinel, or ZnO/ZnAl₂O₄ i.e. zinc oxide supported on zinc aluminium spinel.

For instance, the ZnO/(CeO₂/ZrO₂) MeOH synthesis catalyst is a catalyst comprising ZnO supported on a CeO₂/ZrO₂ carrier.

Now with respect to the oxygenate conversion catalyst, a zeolite with a framework having a 10-ring pore structure means a pore circumference defined by 10 oxygen atoms.

A 1-D pore structure means zeolites containing non-intersecting pores that are substantially parallel to one of the axes of the crystal. The pores preferably extend through the zeolite crystal.

The three letter code, e.g. *MRE, for structure types are assigned and maintained by the International Zeolite Association Structure Commission in the Atlas of Zeolite Framework Types, which is at http:// www.iza-structure.org/databases/ or for instance also as defined in "Atlas of Zeolite Framework Types", by Ch. Baerlocher, L.B. McCusker and D.H. Olson, Sixth Revised Edition 2007.

It would be understood that the term "ZSM-48" may be used interchangeably with the term "EU-2".

In an embodiment, in step a) the pressure is in the range 15-100 bar, such as 20-80 bar or 30-60 bar, and the temperature is in the range 240-400°C, such as 240-360°C, or 300-360°C. At these conditions, there is high activity for methanol conversion, and the content of aromatics in the olefin product stream is further reduced, while at the same time the weight ratio of higher olefins (C3-C8 olefins) to lower olefins (ethylene) is increased. A higher proportion of C3-C8 olefins is desirable for being able to withdraw a significant amount as C3-olefin product as well as for further downstream oligomerization of in particular the C4-C8 olefins into jet fuel. Furthermore, the lower the temperature, the lower the propane content in the olefin product stream, which is desirable for easier separation of propylene being produced.

Suitably, the pressure is said 15-100 bar, such as 30-60 bar, and the temperature is 240-400°C such as 300-360°C.

The obtained olefin product stream exiting step a) is thereby essentially free of aromatics, ethylene (C2=) and propane, yet with significant content of propylene e.g. 10-40 wt% or 10-35 wt%, for instance about 15 wt%, 20 wt% or 30 wt%, and even higher content of higher olefins (C4= - C8=) e.g. 40-60 wt% such as about 50 wt% and optionally a significant content of isoparaffins e.g. 10-15 wt%. For instance, with step a) being conducted with a 1-D zeolite such as ZSM-48, the content of propylene (C3=) in the olefin product stream may be about 15-25 wt% at 320°C and about 30-40 wt% at 360°C. At the same time, the content in the olefin product stream of desirable C4-C8 oleifns is kept at about 40-60 wt% or higher, and the content of in some instances, the less desirable aromatics, below 10 wt% and even below 5 wt%.

It would be understood that the term "temperature" means the C-MTO reaction temperature in an isothermal process, or the inlet temperature to the C-MTO in an adiabatic process. The same applies for the temperature in the OLI and/or HYDRO step.

In an embodiment, step a) is conducted isothermally.

In an embodiment, step a) is conducted adiabatically. The adiabatic temperature rise, defined as the difference between outlet and inlet temperature, is for instance 40-100°C.

In an embodiment, the dual-function catalyst comprising the MeOH synthesis catalyst and the oxygenate conversion catalyst in step a) comprises a binder. The dual-function catalyst is suitably shaped by combining the MeOH synthesis catalyst and the oxygenate conversion catalyst with the binder, and then forming a catalyst body, e.g., pellets or extrudates. Prior to shaping, the zeolite of the oxygenate conversion catalyst may optionally be treated with a phosphoric reagent to create a zeolite having a phosphorous component between 0.5 and 5 wt % of the treated zeolite. Phosphorus may also be added by impregnating the shaped catalyst. As suitable P compounds may be mentioned aqueous mixtures of phosphoric acid, ammonium dihydrogen phosphate and/or diammonium hydrogen phosphate. The phosphorous provides stability to the catalyst. The binder is used to confer hardness and strength on the catalyst. Binders include alumina, aluminum phosphate, silica, silica-alumina, zirconia, titania and combinations of these metal oxides, and other refractory oxides, and clays such as montmorillonite, kaolin, palygorskite, smectite and attapulgite. A preferred binder is an aluminum-based binder, such as alumina, aluminum phosphate, silica-alumina and clays.

The MeOH synthesis catalyst and the oxygenate conversion catalyst may be present in the dual-function catalyst in different amounts, spanning MeOH synthesis catalyst to oxygenate conversion catalyst ratios between 10:1 and 1:10 by weight, such as between 5:1 and 1:5, such as between 2:1 and 1:2. If a binder is applied to shape the dual-function catalyst, e.g. by extrusion, the binder may typically constitute between 5 and 50 % by weight of the shaped catalyst body.

In an embodiment, the dual-function catalyst in step a) contains 10-90 or 20-80 wt% MeOH synthesis catalyst, 10-90 or 20-80 wt% oxygenate conversion catalyst including the binder suitably constituting 5-50 wt% of the oxygenate conversion catalyst, the binder suitably comprising an alumina component such as either alumina itself or a silica-alumina. Upon providing the dual-function catalyst, it would be understood that the above wt% sum up to 100 wt%. For instance, the dual-function catalyst may consist of 40 wt% MeOH synthesis catalyst, 60 wt% oxygenate conversion catalyst including the binder. For instance also, the process may comprise providing the dual-function catalyst with a binder, suitably by mixing the dual-function catalyst, i.e. MeOH synthesis catalyst and oxygenate conversion catalyst, with the binder, such that the dual-function catalyst contains up to said 20-60 wt% MeOH synthesis catalyst, 20-60 wt% oxygenate conversion catalyst, and the binder; the binder suitably comprising an alumina component such as either alumina itself or a silica-alumina, thus forming a silica-alumina binder. Again, the above wt% sum up to 100 wt%. As an example, the dual-function catalyst is 40 wt% MeOH synthesis catalyst, 40 wt% oxygenate conversion catalyst, and 20 wt% alumina.

Prior to for instance mixing with zeolite and binder, the MeOH synthesis catalyst may be calcined, or calcined and, subsequently reduced, e.g., in a hydrogen-containing atmosphere.

It would be understood that the wt% of oxygenate conversion catalyst including the binder means the wt% of the zeolite with respect to the oxygenate conversion catalyst weight, in which the oxygenate conversion catalyst comprises the zeolite e.g. ZSM-48, and the binder.

It would be understood that wt% of the dual-function catalyst, i.e. MeOH synthesis catalyst and oxygenate conversion catalyst, and the binder, means the wt% with respect to the dual-function catalyst. The binder, containing for instance alumina, conveys therefore the dual-function catalyst having the alumina content of for instance said 20 wt%.

It would also be understood, that for the purposes of the present application, the term "binder" is also referred to as "matrix binder" or "matrix/binder" or "binder/matrix".

The binder confers hardness and strength of the catalyst in the C-MTO step a). However, it has now been found that the use of a binder in the catalyst comprising an alumina component, also conveys the undesired effect of MeOH/DME-cracking in step a) when operating at temperatures above 360°C, thereby producing methane as an undesired by-product. The methane needs to be disposed of, e.g. by burning or flaring, which increases the carbon footprint of the process and plant. Further, the yield of the desired olefin product stream comprising i.a. higher olefins is reduced, as some of the feed is converted to the undesired methane by-product instead. It would be understood, that in the course of the oxygenate-to-olefin reaction, methanol may be initially converted to DME by methanol dehydration.

Hence, it turns out that the binder of the catalyst at the low C-MTO temperatures of an embodiment of the present invention, particularly where the catalyst comprises a zeolite with a framework having a 10-ring pore structure, in which said 10-ring pore structure comprises: (a) a unidimensional (1-D) pore structure, said unidimensional (1-D) pore structure is selected from any of *MRE (ZSM-48), MTT (ZSM-23), TON (ZSM-22), or combinations thereof; the pressure is 1-25 bar, such as 2-25 bar, and the temperature is 240-360°C such as 300-360°C, does not promote MeOH/DME cracking so the undesired methane is not produced, thereby enabling an increase in yield of the desired olefin product stream as explained above. Without being bound by any theory, it is believed that by the present invention, the DME quickly reacts to olefins before cracking into methane.

The invention provides thereby the benefits associated to having a binder, incl. better stability of the catalyst, while at the same time eliminating its disadvantages, namely the promotion of MeOH/DME cracking into undesired methane.

In an embodiment, in step a) the MeOH synthesis catalyst and the oxygenate conversion catalyst are shaped as individual catalysts, e.g., as described above, and subsequently provided in the conversion reactor (C-MTO reactor) as a physical mixture.

The dual-function catalyst comprising the MeOH synthesis catalyst and the oxygenate conversion catalyst are thus physically mixed and charged, i.e. loaded, to the conversion reactor as a physical mixture forming the fixed bed in the conversion reactor. Hence a fixed bed is formed which consists of individual bodies of the MeOH synthesis catalyst and the oxygenate conversion catalyst, respectively. An even distribution of the catalyst is thereby provided, which renders a better performance than providing a stepwise approach by for instance providing first a fixed bed of MeOH synthesis catalyst and subsequently a fixed bed of oxygenate conversion catalyst. For instance, an even distribution of the catalyst provides better control of the adiabatic temperature increase in the C-MTO reactor.

In an embodiment, the zeolite of the oxygenate conversion catalyst has a silica-to-alumina ratio (SAR) of up to 500.

The oxygenate conversion catalyst in step a) may be prepared by standard methods in the art, for instance as disclosed in US 4,476,338 for ZSM-48. Suitably, there is mixing of the final catalyst with a binder/matrix, such as in a catalyst that contains up to 50-80 wt% zeolite in a matrix/binder comprising an alumina component such as a silica-alumina matrix binder.

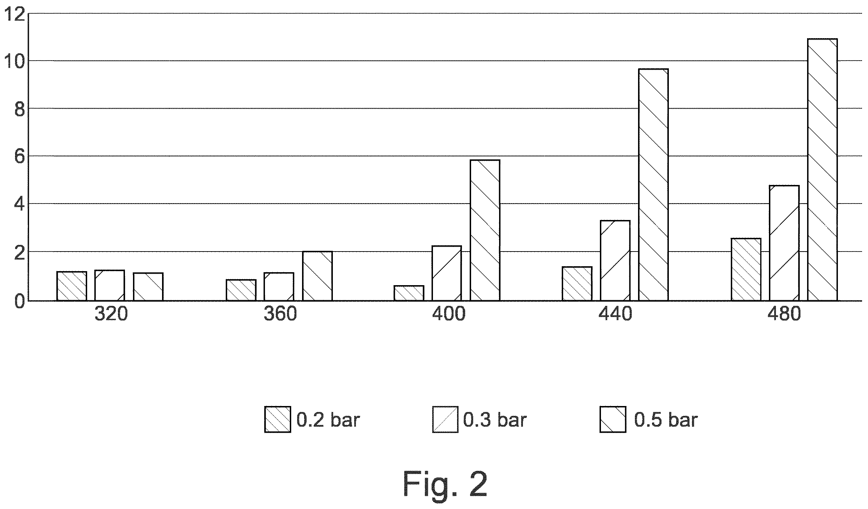

As already recited, the pressure in step a) is 15-100 bar. The pressure may be 20, 40, 60 or 80 bar. High pressure increases the rate of methanol formation. At the same time methanol is formed, it starts reacting over the oxygenate conversion catalyst, e.g., ZSM-48, forming primarily olefins. However, also other hydrocarbons are formed, paraffins, isoparaffins, naphthenes and aromatics. The rate of formation of aromatics increases with temperature and MeOH concentration (partial pressure) as illustrated in the appended Fig. 2. It is seen that at low temperature, below about 360°C little or no increase in aromatics formation is observed as MeOH partial pressure increases, whereas, at temperatures of about 400°C there is a dramatic increase in the formation of aromatics as P_{MeOH} increases.

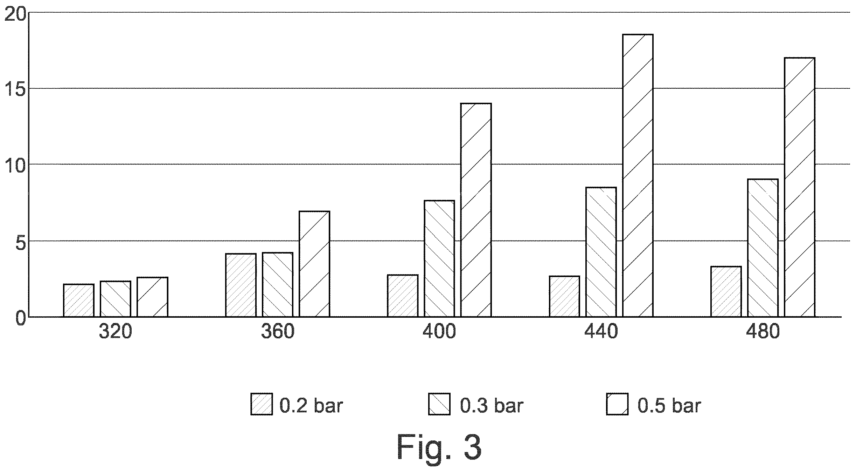

As mentioned, not only the aromatics selectivity is affected by the MeOH partial pressure: the selectivity to both i- and, in particular, n-paraffins are sensitive to both temperature and MeOH partial pressure, as shown in appended Fig.3. Especially the amount of propane increases with increasing temperature and P_{MeOH} and may account for more than half of the total n-paraffins. The formation of paraffins, propane in particular, is known to be associated with the formation of aromatics. Thus, limiting the formation of often undesirable aromatics also provides losses due to paraffin formation at a minimum.

As aromatics may be undesirable, the concurrent formation of MeOH and further reaction of MeOH to form olefins in accordance with the present invention, helps maintaining P_{MeOH} low in the catalyst bed throughout the entire length of the C-MTO reactor, which suitably operates as an adiabatic reactor. The invention enables therefore to obtain a higher selectivity to olefins and suppress the formation of undesired byproduct such as aromatics and paraffins.

Furthermore, by controlling the proportion of MeOH and oxygenate conversion catalyst in the bifunctional catalyst, e.g. within the ratios of 10:90 and 90:10 by weight, P_{MeOH}, such as 20:80 and 80:20 by weight, P_{MeOH} may be controlled in an adequate manner so as to ensure a low methanol partial pressure throughout the entire catalyst bed.

Catalysts suitable for MeOH synthesis typically require high pressure do drive the conversion of synthesis gas towards MeOH. Typically, pressure ranges are between 60-120 bar. High pressure, e.g., 30-60 bar enables a better match with the pressure of the subsequent oligomerization or OLI/HYDRO, as also explained farther below, and thereby significant compression energy savings. The invention provides, therefore, also a process whereby it is now possible to closely match the pressure of the C-MTO with the pressure of the subsequent oligomerization or OLI/HYDRO, while still maintaining high conversion and an olefin product stream which is ideal for subsequent oligomerization and/or OLI/HYDRO.

At the low temperatures of the C-MTO (i.e. in step a)), suitably 240-360°C, with an oxygenate conversion catalyst being a zeolite having a 1-D pore structure such as ZSM-48, the partial pressures of the feed, e.g. methanol (P_{MeOH}), do not play a decisive role in terms of selectivity to aromatics, provided that the P_{MeOH} is low enough, i.e. significantly lower than 1 bar and more suitably, by reference to Fig. 2, lower than 0.2 bar. Compared with operation at temperatures higher than e.g. 360°C, where P_{MeOH} has a high effect on the amount of aromatics produced, with higher P_{MeOH} significantly generating higher content of aromatics, by the present invention where operation of the C-MTO is conducted at temperatures of suitably 360°C or below, e.g. 320°C, particularly with an oxygenate conversion catalyst having a unidimensional (1-D) pore structure selected from any of *MRE (ZSM-48), MTT (ZSM-23), TON (ZSM-22), or combinations thereof, changing the P_{MeOH} does not show any significant influence, as the content of aromatics is maintained below 2 wt% or below 1 wt% and at similar values regardless of the P_{MeOH}. For instance, with ZSM-48 (EU-2), 440°C, methanol concentration in the feed of 10% (volume basis), P_{MeOH} of 0.3 and 0.5 the content of aromatics is about 3 wt% and 9 wt%, respectively; operating at 400°C, at P_{MeOH} of 0.3 and 0.5 the content of aromatics is about 2 wt% and 6 wt%, respectively. Now, when operating between 320 and 360°C, at P_{MeOH} of 0.3 and 0.5 the content of aromatics is about 1 wt% and 2 wt%, respectively; and when operating at 320°C, at P_{MeOH} of 0.3 and 0.5 the content of aromatics is about 1 wt% at both P_{MeOH}. Thus, the higher the P_{MeOH}, the higher the content of aromatics, yet by reducing the temperature, suitably 360°C or below, it is possible to increase the P_{MeOH} to some degree and thereby the pressure (total pressure), without producing more aromatics. The higher independence of the aromatic content with respect to P_{MeOH} being below 1 bar, such as 0.5 bar or lower, as well as the lower C-MTO temperatures, for instance at temperatures of particularly 360°C or below, such as 350°C or 340°C or 320°C or 300°C, enables also operation at high pressure (total pressure), e.g. 15, 20 or 25, 30, 40 or 60 bar. Not only are these pressures better matched to the downstream operations, e.g. oligomerization or OLI/HYDRO, as mentioned above, but they are also closer to the pressures used in the upstream process, in particular the production of the synthesis gas, which may operate at high pressures, for instance when producing synthesis gas by steam methane reforming. Higher energy savings in terms of lower compression energy is thereby achieved, as is a reduction in equipment size.

Now, by the present invention a higher MeOH partial pressure, i.e. P_{MeOH} above 0.6, such as 1 bar, tends to increase selectivity to aromatics and light paraffins, thereby reducing the selectivity to (desired) olefins. For instance, at P_{MeOH} = 1 bar, aromatics (and light paraffins) selectivity increases even at low temperature such as 320-340C. The present invention enables maintaining the MeOH partial pressure as low as possible throughout the catalyst bed(s) in order to maximize the selectivity to olefins, by applying a dual-function catalyst capable of simultaneously converting the synthesis gas into MeOH and MeOH into olefins, thus constantly maintaining the MeOH concentration at a low level, and thereby reducing the selectivity to aromatics/paraffins and improving selectivity to olefins.

Despite the relatively low temperatures used, i.e. reaction temperatures of 240-360°C e.g. 260-360°C, or 260-340°C, the catalysts are also active in not only suppressing the formation of aromatics which in some instances may be unwanted in the feed to OLI, but also in providing a high selectivity for the higher olefins C3=-C8=, essentially no ethylene formation, significant isoparaffin formation, full conversion, while also showing an extended catalyst lifetime. For instance, ZSM-48, when applied at said low temperatures, converts methanol to an olefin product stream which is ideal for further oligomerization and hydrogenation to jet fuel, particularly SAF in accordance with ASTM as defined above. The fact that the olefin product is essentially free of ethylene and aromatics, the latter in some instances being less desirable, while the yield of C3-C8 olefins is between 70-80%, combined with 10-15% isoparaffins, makes the product an ideal feed for further oligomerization to SAF and optionally also diesel. Surprisingly also, a significant amount of C3 olefins is formed which is simple to separate and withdraw in the process as a valuable chemical product, or suitably for recycling back to the C-MTO (step a)) along with other light hydrocarbons such as propane, ethane and ethylene.

The combination of operating the oxygenate conversion with e.g. ZSM-48 and lower temperature (e.g. 300-360°C) conveys at least three highly beneficial effects:
a) the selectivity to ethylene and aromatics is decreased to below 1 wt% in either case;
b) a significant amount of isoparaffins may be formed, which can be used in the process. Isoparaffins, as well as the C3-C8 olefins, in particular C4-C8 olefins, may also be oligomerized, so that isoparaffins may be formed as a desired by-product. The iso-paraffins may optionally be separated for alkylation to increase octane number and then be incorporated into a gasoline pool, or simply be used as part of the olefin product stream for downstream oligomerization;
c) due to the lower applicable temperature, the overall lifetime (number of cycles) of the catalyst is increased as an effect of the lower dealumination rate (affected by the combination of high temperature and water vapor produced during reaction). Further, the lifetime during each cycle, i.e. cycle time, of the catalyst is substantially increased, which without being bound by any theory, is probably an effect of the lower selectivity to aromatics due to less hydrogen transfer reactions. High catalyst longevity in terms of both overall lifetime (number of cycles) and cycle time, is highly important for enabling its use in actual commercial applications;
d) once again, the selectivity towards production of propylene is increased, thereby enabling the production, in a simple manner, of highly pure propylene, which is a product normally having a significantly higher market price than jet fuel.

For the purposes of the present application, the terms "catalyst longevity" and "catalyst lifetime" are used interchangeably.

Accordingly, the features of the invention cooperate synergistically to bring about a superior process which is commercially applicable for conversion of carbon oxides to oxygenates to olefins and thereby for the subsequent downstream steps, e.g. oligomerization for producing SAF, while at the same time integrating within the same process the production of highly valuable chemical grade propylene.

While a suitable oligomerization feed may normally have some aromatics, for instance 10-20 wt% aromatics, as well as higher olefins and ethylene, the ideal oligomerization feed is namely substantially free of aromatics and composed of higher olefins, particularly C4-C8 olefins, and preferably as little as possible C2- light fraction, more particularly, free of ethylene. The lower the temperature in the C-MTO, the higher the content of higher olefins and thereby also the ratio of higher olefins to ethylene, i.e. the selectivity to higher olefins. Also, the lower the temperature, the lower the content of ethylene so the olefin product stream is essentially ethylene-free, while the content of isoparaffins increases. Further, by having removed the C3-olefins from the first olefin product stream, the olefin product stream to downstream oligomerization becomes richer in C4-C8 olefins and thereby easier to oligomerize, e.g. by simple dimerization, to the relevant jet fuel range C8-C16.

The oligomerization feed complies with the above ASTM requirements stipulating the 50% SAF blending part to be almost aromatic-free, more specifically that the content of aromatics be limited to below 0.5 wt%. The olefin product stream can be converted into such jet fuel via oligomerization and hydrogenation in a more efficient overall process due to i.a. less recycling in the oligomerization and higher oligomerization yields. In other words, the higher olefins and low selectivity to aromatics and ethylene simplifies separation steps and increase overall yields of the jet fuel.

By using the moderately high pressure of e.g. 20-80 bar, for instance 30-60 bar in the C-MTO, it is possible to further shift the selectivity towards higher olefins. It has namely been found that while higher pressures increase the ratio of higher olefins to lower olefins i.e. higher selectivity to higher olefins, the higher pressures may also decrease the total yield of olefins (i.e. lower conversion of the oxygenate feed to olefins) and also increase the required temperature to achieve full conversion, which in turn creates the risk of less desired cracking reactions taking place. At the pressure range of 30-80 bar, it is now possible to obtain a higher selectivity to higher olefins without requiring increasing the temperatures to e.g. above 360°C for achieving full conversion, thereby also reducing the occurrence of cracking reactions. By conducting the process at pressure at 20 bar or higher, it is also possible to provide an amount of diluent "heat sink" for the exothermal reaction. The invention enables a high flexibility in the selection of pressures, for instance at the higher end of the pressure range, such as 40-80 bar, as also explained farther above.

At the same time, reducing the temperature in the C-MTO to for instance 320°C or 300°C or lower, despite this in principle implying a reduction in methanol conversion, in fact still maintains the oxygenate conversion at close to 100%, while at the same time maintaining, in particular for an oxygenate conversion catalyst having a zeolite with 1-D pore structure, the content of ethylene below 1 wt%, and the content of propylene at a significant high level, e.g. 25 wt% or higher, for subsequent separation as valuable product, or for recycling ethylene and at least a portion of the propylene back to the C-MTO, as explained above. Further, the content of aromatics is also maintained at below 1 wt%. In particular, having a low content of ethylene and high content of propylene is highly desirable.

Suitably, in step a) there are at least two C-MTO reactors operating in parallel to allow for continuous operation in at least one C-MTO reactor while regenerating at least one other C-MTO reactor. The regeneration procedure includes a step where the C-MTO catalyst is contacted with an oxygen containing stream.

In an embodiment, prior to passing said syngas over said dual-function catalyst (in step a)), the syngas passes over an upstream bed comprising a methanol synthesis catalyst, i.e. an additional methanol synthesis catalyst arranged farther upstream. In a particular embodiment, the methanol synthesis catalyst is different from the MeOH synthesis catalyst of step a). For instance, the upstream bed is provided with a Cu/Zn/Al catalyst in a methanol pre-converting reactor, e.g. a pre-converting reactor arranged upstream the C-MTO reactor, or any other conventional catalyst for the synthesis of methanol. In another particular embodiment, the methanol synthesis catalyst is the same as the MeOH synthesis catalyst of step a), suitably arranged upstream in the same reactor (C-MTO reactor). Thereby, the oxygenate e.g. methanol, is pre-produced in the fixed bed reactor of step a) for feeding it to a downstream fixed bed comprising the dual-bed catalyst.

In an embodiment, the process further comprises directing a portion of the olefin product stream, suitably comprising light hydrocarbons such as ethane/ethylene, propane/propylene and butanes/butene, to step a), such as to a point upstream said dual-function catalyst, suitably in between said upstream bed comprising methanol synthesis catalyst and said fixed bed comprising a dual-function catalyst. Thereby, the portion of the hydrocarbons acts as a heat sink to reduce the exothermicity in step a), which is particularly relevant since the catalyst is arranged as a fixed bed and thus a significant adiabatic temperature increase takes place. This temperature rise can be 100°C or higher, which if kept unchecked, may significantly damage the catalysts and bring the reaction temperature to a high level where e.g. there is a significant production of aromatics. Suitably, said portion of the olefin product stream is added immediately downstream the upstream bed comprising methanol synthesis catalyst. At this point of addition, the oxygenate(s) being produced is mixed with the hydrocarbons for reducing the exothermicity in the downstream bed where the oxygenate(s) is converted to olefins. It would be understood that the term "ethane/ethylene" means ethane and/or ethylene; the term "propane/propylene" means propane and/or propylene; the term "butane/butene" means butane and/or butene. For instance, ethane and/or ethylene means ethane, ethylene, or a combination thereof.

The syngas may also be combined with an inert diluent, such as nitrogen or carbon dioxide or a light paraffin such as methane, thereby reducing the exothermicity in step a), which again is particularly preferred when the catalyst is arranged as a fixed bed, or it may be combined with C2+ hydrocarbons, e.g., by recycling non-condensable hydrocarbons from one or more product separation steps following the C-MTO step and/or the OLI step.

Suitably, the diluent is a combination of said first recycle stream and said inert diluent.

The provision of the first recycle stream gives other advantages. By mixing minor amounts of hydrocarbons, including ethylene, propylene and optionally also higher olefins in the first recycle stream with the syngas feed or with the oxygenate being produced in said first methanol synthesis catalyst, the stability, i.e. the life time, of the catalysts, in particular the downstream catalyst comprising a zeolite such as ZSM-48, is significantly increased. In particular, recycling propylene and light olefins allows decreasing the inlet temperature in the reactor in step a), suitably in the downstream catalyst, by promoting the kick-off or initiation of the oxygenate (e.g. methanol) conversion, which results in an increased yield of higher olefins and further increases the catalyst life time. As recited earlier in connection with 1-D zeolite types, the lower the temperature, the lower the content of aromatics and lower olefins being produced, thus the higher the ratio of higher to lower olefins. Lower temperatures in the conversion of oxygenate(s) e.g. 320°C, enable also operation at the higher pressure range, e.g. 30-60 bar or higher, which may be also advantageous by i.a. enabling a higher throughout in the fixed bed reactor of step (a) and reduction of equipment size in the plant for producing SAF.

In a particular embodiment, said portion of the olefin product stream is a first recycle stream comprising C2-C3 olefins or a C3 olefin product stream (propylene stream, i.e. rich in propylene, which is withdrawn from said olefin product stream; and wherein said first recycle stream is produced by the process further comprising, prior to step b):
- conducting the olefin product stream to a first separation step and withdrawing therefrom a liquid hydrocarbon fraction comprising at least 50 wt% of the C3-olefins contained in said olefin product stream, and withdrawing a gaseous fraction comprising C2-C3 olefins (C2=-C3=), suitably also comprising methane, ethane, propane, carbon monoxide, carbon dioxide and hydrogen, as said recycle stream;
- conducting the liquid hydrocarbon fraction to a fractionation step and separating therefrom an C3 olefin product stream.

Thereby, from the fractionation step an olefin product stream which is free of C3-olefins is withdrawn, prior to conducting the oligomerization step (b).

Suitably, said liquid hydrocarbon fraction comprises at least 75 wt%, such as at least 95 wt% of the C3-olefins contained in said olefin product stream. Accordingly, at least 75%, such as at least 90% of the propylene is retained in the liquid hydrocarbon fraction at this point.

The invention enables obtaining a C3 olefin product stream of high purity C3, suitably at least 90 vol% pure: containing at least 90 vol% propylene, for instance at least 93 vol% propylene corresponding to chemical grade propylene, or for instance at least 99.5 vol% corresponding to polymer grade propylene. Thereby, the present invention enables by simple means the production from syngas of a chemical grade or polymer grade C3 olefin product stream as well as an olefin product stream which is highly suitable for downstream oligomerization into SAF. Hence, by the invention it is now also possible to produce SAF or jet fuel components by further oligomerization and hydrogenation, as recited farther below, together with the high purity propylene; i.e. the production of chemical grade propylene and SAF are integrated in a single process and plant. Furthermore, diesel may also be produced.

The process of the invention allows for easy separation of high purity propylene which can be used for chemicals and/or polymers, as it has been found that the olefin product stream from the further conversion of the oxygenate(s) in step a) not only is essentially free of ethylene, but may also be essentially free of propane, which enables simple separation of the propene (propylene) from the olefin product stream. Thereby, polymer grade propylene is provided by simple separating it from the olefin product stream e.g. via simple gas separation such as flash distillation. Propylene is of high commercial importance as a raw material building block and normally, such chemical grade propylene is produced separately, e.g. in a refinery plant, by steam cracking of a hydrocarbon feed such as naphtha, whereby a mixture of propylene and propane is generated, thus requiring a more complicated and expensive separation. For instance, steam cracking requires addition of steam and heating to reaction temperatures of about 850°C, whereby a number of light olefins are formed incl. propylene.

Surprisingly also, it has been found, that an oligomerization feed low in propylene is beneficial for the OLI reaction: the effect of removing propylene from the feed to the oligomerization step (i.e. the olefin product stream) improves the yield in this OLI-step of the desired branched C8-C16 fraction for jet fuel. Without being bound by any theory, propylene appears reactive for oligomerization, yet not selective, as it competes with or removes the active centres of the higher olefins in the olefin product stream that is fed to the downstream oligomerization and which active centres are highly selective for oligomerization.

In an embodiment, still subsequent to the C-MTO step a) yet prior to OLI step b), the first separation step further comprises withdrawing a water stream and the separation is conducted in a separation unit at 20-80°C, e.g. about 25°C, and 5-50 bar, such as 10-30 bar, e.g. 15 bar.

The separation is conducted in a separation unit. The separation unit is suitably conducted in a 3-phase separator.

The olefin product stream is therefore cooled in the separation unit, e.g. down to 25°C, while adjusting the pressure to the range 5-50 bar, such as 10-30 bar, e.g. 15 bar. This causes the olefin product stream to separate into: said gaseous fraction containing some propylene and which may also contain propane, ethene, ethane, methane, carbon monoxide, carbon dioxide and hydrogen, said liquid hydrocarbon fraction and liquid water (aqueous phase). For instance, the gaseous fraction has a low concentration such as less than 3 vol% or such as less than 1.5 vol% or such as less than 0.5 vol% or such as less than 0.25 vol% of ethene (ethylene) and a low concentration such as less than 1.5 vol% or such as less than 1 vol% or such as less than 0.5 vol% or such as less than 0.25 vol% of propane. The temperature and pressure in the separation unit is adjusted in such a way that at least 50%, preferably at least 75%, more preferably at least 90% of the propylene is retained in the liquid hydrocarbon fraction at this point.

The liquid water (aqueous phase) is removed from the olefin product stream produced in step a), since its presence may be undesirable when conducting the downstream oligomerization.

The boiling point of propylene at normal pressure is about -47°C, which is impractically low for distillation. However, at for instance 10 bar and 15 bar, the boiling point of propylene is 20°C and 34°C, respectively. The next higher boiling component of the C-MTO products, apart from propane which is present in an acceptable low concentration for at least chemical grade propylene, is isobutene. This compound has a boiling point of e.g. 84°C at 15 bar, which thus allows for an easy separation of a propylene-rich gaseous phase and a liquid phase rich in higher olefins, particularly C4-C8 olefins, suitable for further conversion to jet fuel.

Accordingly, in an embodiment, the fractionation step is a flash step being conducted in a flashing unit, such as a flash distillation unit, at 20-80°C, and 5-50 bar, such as 10-30 bar.

The aqueous phase is withdrawn from the separation unit leaving a condensed hydrocarbon mixture i.e. said liquid hydrocarbon fraction. This mixture is transferred to e.g. a flash distillation unit and heated to e.g. 34°C at a pressure of e.g. 15 bar, whereby propylene distills off and is collected. The resulting C3 olefin product is then of high purity, e.g. at least 93 vol% propene (chemical grade) and may even be polymer grade propene (≥ 99.5 vol% propene), as already described.

In an embodiment, the process further comprises:
- separating from the olefin product stream, an isoparaffin stream.

Hence, isoparaffins may be formed as a desired by-product. For instance, the isoparaffin stream may be separated for alkylation to increase octane number and then be incorporated into a gasoline pool.

By the invention, the process comprises the step:
b) passing at least a portion of the olefin product stream, suitably after separating said C3-olefin product stream for thereby forming said olefin product stream, through an oligomerization step over an oligomerization catalyst, and optionally subsequently conducting a separation step, for thereby producing an oligomerized stream.

Accordingly, the condensed hydrocarbon mixture from the flashing unit, after removal of most of the propylene, thus resulting in the olefin product stream, is conducted to the oligomerization step b) by feeding it to an oligomerization reactor containing an oligomerization catalyst.

The isoparaffins, as well as C4-C8 olefins in the olefin product stream, may also be oligomerized. Hence, the invention enables in instances where having aromatics in feed to oligomerization are less desirable, that in a way, instead of having aromatics as by-product, isoparaffins are now provided as a desired product, which may optionally be separated for use as alkylation feed to increase octane number of gasoline optionally also produced in the process. The provision of the isoparaffin stream separation step increases also flexibility in the selection of zeolites structures used in the oligomerization step.

In an embodiment, said oligomerization step b) comprises subsequently conducting a separation step; withdrawing therefrom a second recycle stream, said second recycle stream suitably being a stream rich in C8-olefins, suitably also comprising at least 50 wt% C8-olefins, and also comprising lower hydrocarbons than C8, for instance lower olefins than C8-olefins such as C5-C7 olefins, and n- and i-paraffins; and directing it to step a), such as to a point upstream said dual-functional catalyst, suitably between said upstream bed comprising methanol synthesis catalyst, and said fixed bed comprising a dual-function catalyst.

The recycle of, among others, C8-olefins, serves as heat-sink in step (a), as well as a co-feed in this step. The recycled compounds, being relatively heavy, serve therefore as a good heat sink in the C-MTO reactor which is highly exothermic, and some may further react to olefins. Increase integration of the process and plant as well as increased carbon utilization is thereby achieved.

The oligomerization step (step b) is preferably conducted in an oligomerization reactor by conventional methods including the use of an oligomerization catalyst such as a solid phosphoric acid ("SPA"), ion-exchange resins or a zeolite catalyst, for instance a conventional *MRE, BEA, FAU, MTT, TON, MFI and MTW catalyst, at a pressure of 30-100 bar, such as 50-100 bar, and a temperature of 100-350°C. The products from the oligomerization reaction may be subsequently separated in said separation step, such as distillation, thereby also withdrawing a lighter hydrocarbon stream such as naphtha, which comprises C5-C7 hydrocarbons, and the oligomerized stream, which comprises C8+ hydrocarbons.

By the invention, the process further comprises the step:
c) passing at least a portion of the oligomerized stream through a hydrogenation step over a hydrogenation catalyst, and optionally subsequently conducting a separation step, for thereby producing a hydrocarbon stream comprising hydrocarbons boiling in the jet fuel range, optionally also comprising hydrocarbons boiling in the diesel fuel range.

The hydrocarbon product stream from the oligomerization step b) is primarily branched olefins in the C8-C16 range but may also contain e.g. C4-C7 olefins, n- and iso-paraffins, naphtenes and aromatics. The lower boiling fraction may be recycled over the oligomerization reactor as a third recycle stream in the process ("OLI recycle") in order to increase the overall yield of C8-C16 olefins. The higher boiling fraction is finally conducted to hydrogenation step in a hydrogenation reactor together with H₂ to saturate the olefins and optionally also to hydrogenate any aromatics to naphthenes. The product from the hydrogenation reactor is useful as jet fuel and as a jet fuel component and is the other product of the process of the present invention. The resulting jet fuel is SAF. Accordingly, in a particular embodiment, the hydrocarbon stream comprising hydrocarbons boiling in the jet fuel range is SAF, i.e. a sustainable aviation fuel in compliance with ASTM D7566 and ASTM D4054.

Suitably, the partial pressure of hydrocarbons in a hydrogenation reactor of step c) is not higher than the pressure in the oligomerization reactor of step b).

The hydrogenation step may be conducted by methods, including under the presence of hydrogen, the use of a hydrotreating or hydrogenation catalyst, for instance a catalyst comprising one or more metals, e.g. Pd, Rh, Ru, Pt, Ir, Re, Cu, Co, Mo, Ni, W or combinations thereof, at a pressure of 1-100 bar such as 60-70 bar, and a temperature of 0-350°C, such as 50-350°C. The C8+ hydrocarbons of the oligomerized stream are thereby saturated to form the corresponding paraffins. These may be subsequently separated in a separation step, for instance a distillation step, whereby any hydrocarbons boiling in the diesel range are withdrawn and thereby separated from the hydrocarbons boiling in the jet fuel range i.e. jet fuel.

Yet, now more specifically, it has also been found, that a proper choice of hydrogenation catalyst can strongly affect the content of aromatics in the SAF product. Thus, if an aromatics-free SAF product is desired, a Ni-based hydrogenation catalyst such as one based on nickel on alumina (Ni/Al₂O₃) can be used. Such catalyst is capable of saturating most or all aromatic compounds. The aromatics are thereby converted to naphthenes, which happen to be desirable compounds in SAF. The aromatics from the C-MTO step a) are alkylated in the OLI step b) so that they come out in the C12-C14 range. Thus, in the oligomerization step, aromatics are to some extent alkylated by olefins. This increases the carbon number of the aromatics. These aromatics are then hydrogenated to their corresponding naphthenes in the hydrogenation step. Thereby, increased flexibility in the process is achieved, as one is not confined to strictly producing an olefin product stream with low aromatics for the subsequent oligomerization. The C-MTO step (step a) may thus be conducted at a wider range of temperatures, for instance at temperatures of 360°C or higher, such as 400°C or 450°C, where more aromatics are produced, without this being detrimental for the subsequent production of jet fuel. If, on the other hand, aromatics are desired as a component of the SAF product, another type of hydrogenation catalyst can be used, such as a Cu-based one, e.g. Cu/ZnO/Al₂O₃ or Cu/ZnAl₂O₄. Such Cu-based hydrogenation catalysts will efficiently saturate the olefins while most of the aromatics are left unchanged.

Accordingly, in an embodiment, the hydrogenation catalyst in step c) is a Ni-based hydrogenation catalyst, i.e. a hydrogenation catalyst containing Ni as the active metal, suitably a supported Ni catalyst having a Ni content of 1-25 wt% such as 10-15 wt%, based on the total weight of the catalyst, and wherein the support is selected from alumina, silica, titania and combinations thereof. In another embodiment, the hydrogenation catalyst in step c) is a Cu-based hydrogenation catalyst, i.e. a hydrogenation catalyst containing Cu as the active metal, suitably a supported Cu-based catalyst having a Cu content of 10-75 wt%, suitably 12-38 wt% based on the total weight of the catalyst as in applicant's co-pending patent application PCT/EP2021/082821, and wherein the support is selected from alumina, zinc oxide, zinc aluminum spinel, silica, titania and combinations thereof.

The hydrogenation step, as recited above, is suitably conducted at a pressure of 1-100 bar and a temperature of 0-350°C.

In an embodiment, the entire oligomerized stream passes through the hydrogenation step. As used herein, the term "entire oligomerized stream" means at least 90 wt% of the stream.

It has also been found that the product ratio of propylene to jet fuel (P/JF) of the process can be varied by adjusting the inlet temperature to the C-MTO reactor in step a). Thus, at e.g. a temperature of e.g. 300°C, P/JF is approximately 0.25 while at a temperature of 400°C or 360°C, P/JF is approximately 0.67 or 0.5-0.6, respectively. Surprisingly, the gaseous propylene-rich fraction from e.g. a flashing unit as described above, is at least 93 vol% propylene at these temperatures and at all temperatures in between, suitably in the range 300-360°C, or even lower temperatures. This propylene purity qualifies for what is called chemical grade propylene and which is of much higher value than lower grades of propylene. Thus, the invention allows for co-production of high grade propylene which has normally a higher value than jet fuel. The chemical grade propylene is easily obtained without further purification, allowing for significant savings both in terms of CAPEX and OPEX (capital expenditure and operating expenditures, respectively). It is a considerable advantage to be able to tune the product distribution of propylene and jet fuel in the process/plant, to comply with the ever-changing product demand from the market.

In an embodiment of the invention, the oligomerization step (step b) and hydrogenation step (step c) are combined in a single hydro-oligomerization step (OLI/HYDRO), e.g. by combining the steps in a single reactor. In other words, by passing at least a portion of the olefin product stream trough an oligomerization step and hydrogenation step which are combined in a single oligomerization-hydrogenation step, and optionally subsequently conducting a separation step, for thereby producing a hydrocarbon stream comprising said hydrocarbons boiling in the jet fuel range, and optionally also diesel. This results in a much simpler process/plant layout.

As used herein, the term "single oligomerization-hydrogenation step" or more generally "single step" or "single stage" means a section of the process in which no stream is withdrawn. Typically, a single stage does not include equipment such as compressors, by which the pressure is increased.

Suitably, the oligomerization step is dimerization, optionally also trimerization, i.e. by conducting the oligomerization at conditions suitable for dimerization and/or trimerization. Thereby the single reactor is preferably operated at a relatively low pressure, such as 15-60 bar, for instance 20-40 bar. The oligomerization reaction is very exothermic per oligomerization step and much less heat is produced, since there is only dimerization, optionally also trimerization, instead of higher oligomerization such as tetramerization or even pentamerization. The lower heat produced favors approaching equilibrium, i.e. higher conversion of olefins.

Normally, the oligomerization step converts the olefins to a mixture of mainly dimers, trimers and tetramers or even pentamers; for instance, a C6-olefin will result in a mixture comprising C12, C18, C24 products and probably also higher hydrocarbons. By conducting the oligomerization step at conditions suitable for dimerization, optionally also trimerization, a more selective and direct conversion of the higher olefins (C3-C8 olefins, in particular C4-C8 olefins) to the jet fuel relevant hydrocarbons, namely C8-C16, is obtained. The dimerization and optional trimerization step comprises the use of lower pressures than in conventional oligomerization processes, thereby also reducing compression requirements which translates into higher energy efficiency - due to lower compression energy- as well as reduced costs, e.g. reduced costs of the oligomerization reactor and attendant equipment, as well as reduced operating costs due to less need of separating C16+ olefins otherwise formed in conventional OLI reactors. Accordingly, the pressure of the OLI/HYDRO can be adapted to better match the pressure of the previous oxygenate conversion step.

Moreover, instead of using a dedicated separation such as distillation in the OLI step for separating naphtha (which can be upgraded to a gasoline product) and another dedicated separation in the hydrogenation step for separating diesel from the jet fuel, only one subsequent separation stage, if any, will be needed. Thereby a simpler process for oligomerization and hydrogenation is obtained and consequently also a simpler overall process and plant.

The hydrogenation or H₂-addition is conducted in the same reactor, for instance by adjusting the activity of the hydrogenation component e.g. nickel. In an embodiment, the single oligomerization-hydrogenation step is conducted in a single reactor having a stacked reactor bed where a first bed comprises an oligomerization catalyst, e.g. zeolite catalyst, and a subsequent bed comprises a hydrogenation catalyst.

In another embodiment, the oligomerization-hydrogenation step is conducted by reacting, under the presence of hydrogen, the olefin product stream, e.g. after separating said isoparaffin stream, over a catalyst comprising a zeolite and a hydrogenation metal, such as a hydrogenation metal selected from Pd, Rh, Ru, Pt, Ir, Re, Co, Cu, Mo, Ni, W and combinations thereof, and preferably at a pressure of 15-60 bar such as 20-40 bar, and a temperature of 50-350°C, such as 100-250°C. In a particular embodiment, the catalyst comprises a zeolite having a structure selected from MFI, MEL, SZR, SVR, ITH, IMF, TUN, FER, EUO, MSE, *MRE, MWW, TON, MTT, FAU, AFO, AEL, and combinations thereof, preferably a zeolite with a framework having a 10-ring pore structure i.e. pore circumference defined by 10 oxygens, such as zeolites having a structure selected from TON, MTT, MFI, *MRE, MEL, AFO, AEL, EUO, FER, and combinations thereof. These zeolites are particularly suitable due to the restricted space of the zeolite pores, thereby enabling that the dimerization is favored over larger molecules.

Suitably, the weight hour space velocity (WHSV) of the OLI/HYDRO step is 0.5-6 h⁻¹, such as 0.5-4 h⁻¹.

Lower pressures corresponding to the operating at conditions for dimerization, optionally also trimerization, are in particular 15-50 bar, such as 20-40 bar. This, again, is significantly lower than the pressures normally used in oligomerization, which typically are in the range 50-100 bar.

Conditions that result in a mild hydrogenation are suitably pursued. Particularly suitable catalysts are catalysts comprising NiW, for instance sulfide NiW (NiWS), or Ni such as Ni supported on a zeolite having a FAU or MTT structure, for instance a Y-zeolite, or ZSM-23. The catalyst which is active for oligomerization and hydrogenation may for instance contain up to 50-80 wt% zeolite in a matrix/binder comprising an alumina component. The hydrogenation metal may then be incorporated by impregnation on the catalyst. The hydrogenation metals are selected so as to provide a moderate activity and thereby better control of the exothermicity of the oligomerization step by mainly hydrogenating the dimers being formed as the oligomerization takes place, thereby interrupting the formation of higher oligomers.

Hence, rather than having separate reactors and attendant separation units for conducting oligomerization and subsequent hydrogenation, each with its own catalyst, the present invention enables in a single oligomerization-hydrogenation step the use of less equipment e.g. one single reactor and optionally a single separation stage downstream for obtaining the jet fuel. A more efficient and simpler overall process and plant for the conversion of oxygenates such as methanol to jet fuels, particularly SAF, is thereby achieved.

Suitably, a stream comprising C8-hydrocarbons resulting from cracked C9-C16 hydrocarbons, is withdrawn from said hydrocarbon stream comprising hydrocarbons boiling in the jet fuel range and added to other processes. For instance, the process according to the invention may cooperate with a refinery plant (or process), in particular a bio-refinery, and the stream comprising C8-hydrocarbons is added to the gasoline pool in a separate process for producing gasoline of said refinery. Optionally, a stream comprising C8- hydrocarbons resulting from cracked C9-C16 hydrocarbons, is withdrawn from said hydrocarbon stream comprising hydrocarbons boiling in the jet fuel range and used (recycled) as additional feed stream to the oligomerization step or the single oligomerization-hydrogenation step.

In an embodiment, the process further comprises, prior to step a):
- passing a water and/or steam stream through an electrolysis step for producing a hydrogen stream,
- providing a CO₂-rich stream, suitably containing at least 60 vol.% CO₂;
- optionally passing the CO₂-rich stream through an electrolysis step for producing a stream enriched in CO (thus still also comprising CO₂);
- combining said hydrogen stream with said CO₂-rich stream or with said stream enriched in CO, to form said synthesis gas; said synthesis gas suitably defining a CO₂/CO molar ratio of at least 2, preferably at least 5;
   or
- passing a hydrocarbon feed stream such as a natural gas stream through a steam reforming step, suitably in an electrically heated steam methane reactor (e-SMR), for producing a raw synthesis gas, and optionally
- passing the raw synthesis gas through a secondary reforming unit under the addition of oxygen, for producing said synthesis gas; said synthesis gas suitably defining a CO₂/CO molar ratio of at least 2, preferably at least 5.

Suitably also, the synthesis gas comprises H₂ and the carbon oxide(s) in a molar ratio of at least 3:1.

In particular, when applying electrolysis as for instance disclosed in applicant's co-pending patent application PCT/EP2021/086999 or when utilizing e-SMR as in applicant's WO 2019/228797 A1, a sustainable front-end solution is provided. The power (electricity) required for the electrolysis or for the e-SMR is advantageously supplied from renewable sources, such as wind, solar, hydropower. A process and plant having a low carbon footprint is thereby provided.

In yet another embodiment, as part of the front-end solution, a portion of the CO₂ may be converted to CO. A reactor system and a process may therefore be provided for carrying out reverse water gas shift reaction of a feedstock, here syngas, comprising CO₂ and H₂, to a first product gas comprising CO, where the heat for the endothermic reverse water gas shift reaction is provided by resistance heating, thus in a reverse water gas shift (RWGS) is conducted in electrically heated RGWS reactor, for instance as disclosed in applicant's WO 2021110806 A1.

In an embodiment, the CO₂-rich stream is suitably derived from carbon capture and utilization, CCU, for instance CO₂ being recovered as emission product of a power plant using coal or similar, or CO₂ captured from the air. The CO₂-rich stream may also be derived from a hydrocarbon feed gas, such as hydrocarbon feed gas having a high concentration of CO₂, for instance 20% vol. or more CO₂ such as biogas. Biogas is a renewable energy source that can be used for heating, electricity, and many other operations. Biogas can be cleaned and upgraded to natural gas standards, when it becomes bio-methane. Biogas is primarily methane (CH₄) and carbon dioxide (CO₂), typically containing 60-70% vol. methane. Up to 30% vol. or even 40% vol. of the biogas may be CO₂. Typically, this CO₂ is removed from the biogas and vented to the atmosphere in order to provide a methane rich gas for further processing or to provide it to a natural gas network. By the present invention, the removed CO₂ may be instead utilized as part of the syngas.

In another general embodiment outside of the invention, there is also provided a process for producing a hydrocarbon stream comprising hydrocarbons boiling in the jet fuel range, said process comprising:
- passing a synthesis gas (syngas) stream comprising a carbon oxide and hydrogen over a fixed bed in a conversion reactor, the fixed bed comprising a dual-function catalyst active in the conversion of syngas to the oxygenate(s) methanol (MeOH) and/or dimethyl ether (DME), and the conversion of said oxygenate(s) to an olefin product stream; wherein the dual-function catalyst comprises: a MeOH synthesis catalyst, in which the MeOH synthesis catalyst is a Cu and Cr-free catalyst comprising an oxide of Zn in combination with an oxide of any of Zr, Al, Si, Ti, Ce, La, Ga, In, Mo, Mn, Mg, Y, or combinations thereof; and an oxygenate conversion catalyst comprising a zeolite with a framework having a 10-ring pore structure, in which the 10-ring pore structure comprises a unidimensional (1-D) pore structure selected from any of: *MRE (ZSM-48), MTT (ZSM-23), TON (ZSM-22), or combinations thereof;
- passing at least a portion of the olefin product stream through an oligomerization step over an oligomerization catalyst, and optionally subsequently conducting a separation step, for thereby producing an oligomerized stream;
- passing at least a portion of the oligomerized stream through a hydroprocessing step, such as a hydrogenation step, over a hydroprocessing catalyst; and optionally subsequently conducting a separation step, for thereby producing said hydrocarbon stream comprising hydrocarbons boiling in the jet fuel range, optionally also comprising hydrocarbons boiling in the diesel fuel range.

As used herein, the term "hydroprocessing" means any of hydrotreating, hydrocracking, hydrogenation, or combinations thereof. The hydroprocessing step is conducted in a hydroprocessing reactor i.e. a hydroprocessing unit, comprising a catalyst under the presence of hydrogen. For instance, hydrotreating is conducted over a hydrotreating catalyst for the removal of sulfur, oxygen, nitrogen, and metals from the hydrocarbons; hydrocracking is conducted over a hydrocracking catalyst for the cracking of hydrocarbons; hydrogenation - as already described - is conducted over a hydrogenation catalyst to hydrogenate hydrocarbons.

Hydrotreating and hydrocracking are also well-known in the art. For instance, applicant's WO 2021180805 discloses the associated catalysts and operating conditions.

In another aspect, the invention relates to a plant i.e. a process plant, for conducting the process according to any of the above embodiments. Accordingly, in an embodiment there is provided a plant for conducting the process according to any of the above process embodiments, said plant comprising:
- an oxygenate conversion reactor comprising a catalyst active in the conversion of oxygenates, wherein the oxygenate conversion reactor is arranged to receive a feedstock stream comprising oxygenates and withdraw said first olefin stream, the oxygenate conversion reactor further arranged to operate at temperature of 240-400°C;
- a first separation unit arranged to receive the first olefin stream and withdraw a liquid hydrocarbon fraction comprising at least 50 wt% of the C3-olefins contained in said first olefin stream;
- a fractionation unit arranged to receive the liquid fraction and withdraw said C3 olefin product stream and olefin product stream;
- an oligomerization reactor comprising an oligomerization catalyst, wherein the oligomerization reactor is arranged to receive at least a portion of the olefin product stream and withdraw said oligomerized stream;
- a hydrogenation reactor comprising a hydrogenation catalyst, wherein the hydrogenation reactor is arranged to receive at least a portion of the oligomerized stream and withdraw said hydrocarbon stream comprising hydrocarbons boiling in the jet fuel range.

In another general embodiment of the plant, there is provided a hydroprocessing reactor for treating the at least a portion of the oligomerized stream. Accordingly, there is also provided a plant for conducting the process according to any of the above process embodiments, said plant comprising:
- an oxygenate conversion reactor comprising a catalyst active in the conversion of oxygenates, wherein the oxygenate conversion reactor is arranged to receive a feedstock stream comprising oxygenates and withdraw said first olefin stream, the oxygenate conversion reactor further arranged to operate at temperature of 240-400°C;
- a first separation unit arranged to receive the first olefin stream and withdraw a liquid hydrocarbon fraction comprising at least 50 wt% of the C3-olefins contained in said first olefin stream;
- a fractionation unit arranged to receive the liquid fraction and withdraw said C3 olefin product stream and olefin product stream;
- an oligomerization reactor comprising an oligomerization catalyst, wherein the oligomerization reactor is arranged to receive at least a portion of the olefin product stream and withdraw said oligomerized stream;
- a hydroprocessing reactor comprising a hydroprocessing catalyst, such as a hydrogenation reactor comprising a hydrogenation catalyst, wherein the hydroprocessing reactor is arranged to receive at least a portion of the oligomerized stream and withdraw a hydrocarbon stream comprising hydrocarbons boiling in the jet fuel range.

Any of the embodiments and associated benefits in connection with the process of the invention may be used in connection with the plant of the invention.

Advantages (benefits) of the invention include:
- simultaneously converting synthesis gas in the C-MTO step a) into MeOH and MeOH into hydrocarbons, thereby constantly maintaining the MeOH concentration at a low level, thus reducing the selectivity to aromatics/paraffins and improving selectivity to olefins;
- by removing the propylene from the olefin product stream, better yields of the branched C8-C16 hydrocarbons are obtained, which is desirable, as branched C8-C16 hydrocarbons constitute the main fraction of jet fuel, thereby also SAF. The olefin product stream, containing mainly C4-C8 olefins, or even higher olefins already in the jet fuel range C8-C16, is superior i.e. more suitable for the downstream oligomerization (step b), than an olefin product stream containing mainly C3-C8 olefins;
- by mixing the syngas, or in particular methanol and/or DME being produced in step a), with minor amounts of propylene contained in the gaseous first recycle stream, the stability, i.e. the life time, of the dual-function catalyst is significantly increased. introducing propylene to the feed to the MTO reactor allows for decreasing the inlet temperature in the fixed bed reactor in step a), which results in an increased yield of higher olefins and further increase of the catalyst life time;
- low content of C3-olefin (propylene) in the olefin product stream fed to the oligomerization, as this increases the yield of the desired hydrocarbons boiling in the jet fuel range;
- the product ratio propylene/jet fuel (P/JF) of the overall process can be varied by adjusting the temperature to the fixed bed reactor of the C-MTO step a), suitably the inlet temperature to the reactor. Thus, at a temperature of e.g. 300°C, P/JF is approximately 0.25 while at a temperature of 400°C, P/JF is approximately 0.67. Surprisingly, the gaseous propylene-rich fraction from e.g. a flashing unit when separated prior to the oligomerization step b), is at least 93 vol% propene at both these temperatures in the C-MTO and at all temperatures in between. This propene purity qualifies for what is called chemical grade propylene and which is of much higher value than lower grades of propene. The chemical grade propylene is thus easily obtained without further purification, allowing for significant savings in capital and operating expenses. It is a considerable advantage to be able to tune the product distribution to comply with the ever-changing product demand from the market;
- integration in a simple process of the production of not only SAF, but also diesel and not least chemical grade propylene;
- flexibility in the process with respect to aromatic content in the SAF while at the same time producing chemical grade propylene.
- integration of the OLI step (step b)) and C-MTO step (step a)) by e.g. providing C8 or C8- hydrocarbons, representing relatively heavy compounds, as the second recycle,
and which thereby serve as heat sink in the C-MTO reactor as well as co-feed. In addition, there is increased carbon utilization in the process and plant.

Fig. 1 is a scheme of a process and plant layout according to an embodiment of the invention.

Fig. 2 shows a plot of the content of aromatics (total aromatics, wt%) in the olefin product stream at different temperatures (°C) as well as a function of methanol partial pressures (P_{MeOH} of 0.2-0.5 bar), when converting methanol with a catalyst comprising ZSM-48, in accordance with Example 1.

Fig. 3 provides a similar plot as that in Fig. 2, showing how the selectivity to n-paraffins (wt%) increases with temperature (°C) and methanol partial pressures (P_{MeOH} of 0.2-0.5 bar), when converting methanol with a catalyst comprising ZSM-48, in accordance with Example 1.

Fig. 4 is another plot of the content of aromatics (wt%) in the olefin product stream at different temperatures (°C) and at a higher methanol partial pressure (P_{MeOH}=1 bar), when converting methanol with a catalyst comprising ZSM-48, in accordance with Example 1.

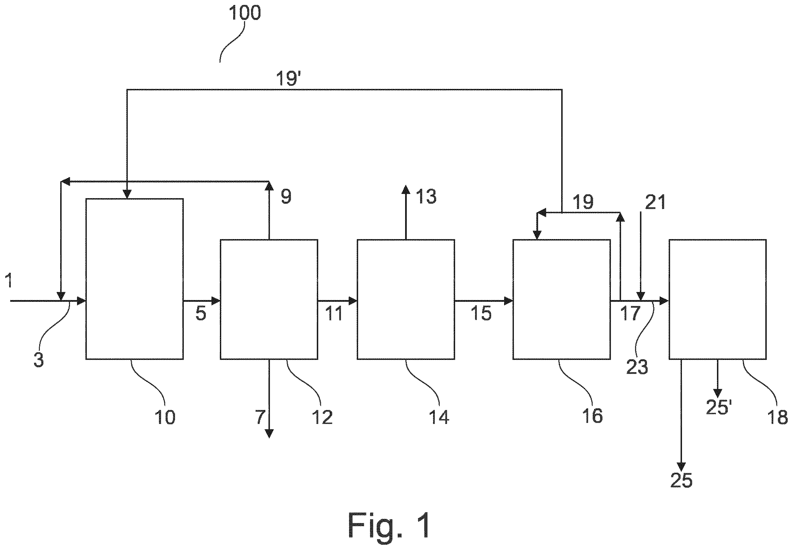

With reference to Fig. 1, schematic layout of process and plant 100 (C-MTJ) for producing jet fuel, in particular SAF, and propylene, from a synthesis gas, is shown. A synthesis gas 1 is provided which is combined with a first recycle stream 9 comprising ethylene and propylene (C2-C3 olefins), which acts not only as diluent to reduce the exothermicity of the C-MTO step in C-MTO reactor 10, but also to enable a lower inlet temperature to the C-MTO reactor 10 as the onset of the reaction for converting oxygenates may then take place at lower temperature. Suitably, the C-MTO reactor 10 is provided with an upstream methanol catalyst bed (methanol synthesis catalyst bed) and a downstream dual-function catalyst bed (not shown). The first recycle stream 9 may be provided to a point in between the upstream methanol catalyst bed and downstream dual-function catalyst bed. After combining with the first recycle stream 9 (from separation unit 12), the syngas feed 3 is passed to the C-MTO reactor 10, thereby producing an olefin product stream 5. The olefin product stream 5 is conducted, optionally after compression, to a first separation step in separation unit 12, suitably a 3-phase separator, and withdrawing therefrom a liquid hydrocarbon fraction 11 comprising at least 50 wt% of the C3-olefins contained in said first olefin product stream 5; as well as a water stream 7 and said first recycle stream 9 as a gaseous fraction containing C2-C3 olefins. The first recycle stream 9 may also comprise methane, ethane, propane, carbon monoxide, carbon dioxide and hydrogen.

The liquid hydrocarbon fraction 11 is conducted to a fractionation step in e.g. a distillation unit, suitably a flashing unit 14, such as a flash distillation unit, thereby easily separating therefrom a C3 olefin product stream 13 having e.g. a propylene purity of at least 93 vol.% (% propene in stream 13), and thus being withdrawn as chemically grade propylene. The olefin product stream 15 now essentially without C3-olefins is also produced, which after optional compression and evaporation, is conducted to an oligomerization step in oligomerization reactor (OLI reactor) 16; thereby producing an oligomerized stream 17, a part of which may be recycled as stream 19 after a subsequent separation (not shown). A second recycle stream 19' suitably rich in C8-olefins, suitably also comprising at least 50 wt% C8-olefins, as well as lower olefins than C8-olefins such as C5-C7 olefins, and n- and i-paraffins; is directed to the C-MTO reactor 10. The recycled compounds, being relatively heavy, serve therefore as a good heat sink in the highly exothermic C-MTO reactor, and some may further react to olefins. The oligomerized stream 17 is then add-mixed with hydrogen 21 and passed as stream 23 through a hydrogenation step (HYDRO) in a hydrogenation reactor 18 (HYDRO reactor) for thereby producing a hydrocarbon stream 25 comprising hydrocarbons boiling in the jet fuel range, particularly as SAF. A diesel stream 25' may also be withdrawn, for instance after a subsequent separation (not shown). Suitably, the OLI and HYDRO step are combined in a single step (OLI/HYDRO) in a single reactor (not shown) having a stacked reactor bed where a first bed comprises an oligomerization catalyst and a subsequent bed comprises a hydrogenation catalyst. The HYDRO reactor may also be provided as another hydroprocessing reactor, such as a hydrotreating reactor or a hydrocracking reactor.

### EXAMPLE 1. Effect of methanol partial pressure in C-MTO

Tests for the conversion of methanol to olefins with ZSM-48 (EU-2) where conducted at WSHV=2 h⁻¹ and methanol concentration in the feed (C_{MeOH}) = 10% (volume basis). Fig. 2 shows the content of aromatics in wt% measured as benzene (B), toluene (T), xylene (X) and ethylbenzene (i..e. total aromatics), at the different temperatures (°C), as well as a function of methanol partial pressures P_{MeOH} at the different temperatures. At a given temperature, for instance at 320°C, each column designates a P_{MeOH} : the column in the left is P_{MeOH}=0.2 bar, the column in the center P_{MeOH}=0.3 bar and the column in the right P_{MeOH}=0.5 bar. At a methanol concentration in the feed of 10%, the pressure (total pressure) at each give partial pressure is respectively: 2 bar, 3 bar and 5 bar.

It is observed that is that at low temperature, from 360°C and below, the MeOH partial pressure does not seem to have any significant influence on the content of aromatics in the resulting olefin stream. The (negative) effect of high MeOH partial pressure in terms of higher production of aromatics, thus appears to decrease drastically at 360°C and more or less vanish at temperatures below about 350°C. This enables an increase in the pressure used for conducting C-MTO, which provides benefits in terms of i.a. higher throughout in the C-MTO and reduction of equipment size in the plant for producing SAF.

As shown in Fig. 2, at the lower temperatures, the content of aromatics is maintained below 2 wt% and at similar values regardless of the P_{MeOH}. For instance, by operating at 400°C, at P_{MeOH} of 0.3 and 0.5 the content of aromatics is about 2 wt% and 6 wt%, respectively. When operating at 360°C, at P_{MeOH} of 0.3 and 0.5 the content of aromatics is about 1 wt% and 2 wt%, respectively. When operating at 320°C, at P_{MeOH} of 0.3 and 0.5 the content of aromatics is about 1 wt% at both P_{MeOH}.

When co-feed of light olefins such as the C3-olefin (propylene) is provided, this enables a further reduction in temperature and, in turn, a further reduction in hydrogen transfer (less generation of e.g. aromatics), higher olefin chain length and, additionally, more freedom with respect to total (or methanol partial) pressure extend ultimate catalyst longevity.

Fig. 3 shows the content of n-paraffins (wt.%) with respect to the temperature (°C): not only the aromatics selectivity is affected by the MeOH partial pressure; the selectivity to both i- and, in particular, n-paraffins are sensitive to both temperature and MeOH partial pressure. Especially the amount of propane increases with increasing temperature and P_{MeOH}, and may account for more than half of the total n-paraffins. The formation of paraffins, propane in particular, is known to be associated with the formation of aromatics.

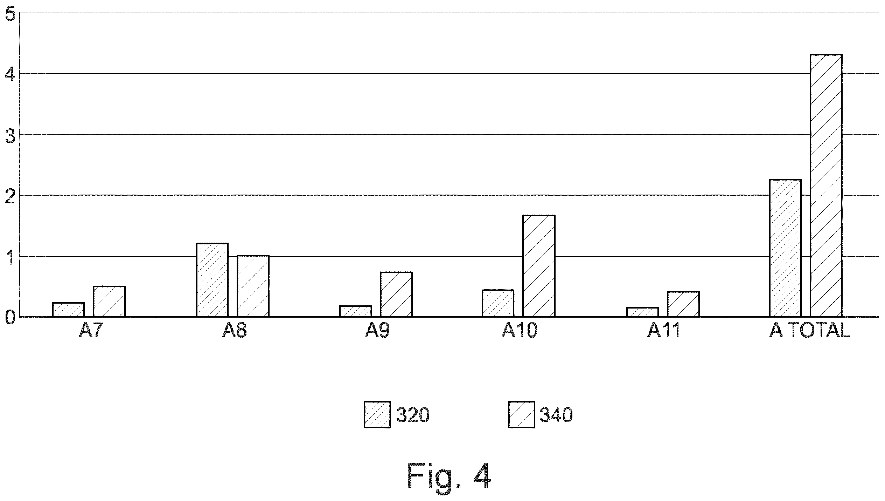

Now turning to Fig. 4, where the MeOH partial pressure is higher, for instance 1 bar, at higher MeOH partial pressure (1 bar), aromatics (and light paraffins) selectivity increases, even at low temperature, here 320-340°C. Fig. 4 shows the content of aromatics (A7:toluene, A8: xylenes, A9: trimethylbenzenes, A10:tetramethylbenzenes, A11: pentamethylbenzenes, and the total content of the aromatics in wt% (A TOTAL)), at the low temperatures 320°C (left-hand columns) and 360°C (right-hand columns). The operating conditions were pressure of 15 bar, methanol concentration 6.7 mol% (i.e. P_{MeOH} = 1 bar), and WHSV of 0.6 h⁻¹.

By maintaining the P_{MeOH} as low as possible throughout the dual-function catalyst bed in the C-MTO reactor (step a) of the invention, the selectivity to higher olefins is significantly increased.

## Claims

1. A process for producing a hydrocarbon stream comprising hydrocarbons boiling in the jet fuel range, said process comprising:
a) passing a synthesis gas (syngas) stream comprising carbon dioxide and hydrogen over a fixed bed in a conversion reactor, the fixed bed comprising a dual-function catalyst active in the conversion of syngas to the oxygenate(s) methanol (MeOH) and/or dimethyl ether (DME), and the conversion of said oxygenate(s) to an olefin product stream; wherein the dual-function catalyst comprises: a MeOH synthesis catalyst, in which the MeOH synthesis catalyst is a Cu and Cr-free catalyst comprising an oxide of Zn in combination with an oxide of any of Zr, Al, Si, Ti, Ce, La, Ga, In, Mo, Mn, Mg, Y, or combinations thereof; and an oxygenate conversion catalyst comprising a zeolite with a framework having a 10-ring pore structure, in which the 10-ring pore structure comprises a unidimensional (1-D) pore structure selected from any of: *MRE (ZSM-48), MTT (ZSM-23), TON (ZSM-22), or combinations thereof;
b) passing at least a portion of the olefin product stream through an oligomerization step over an oligomerization catalyst, for thereby producing an oligomerized stream;
c) passing at least a portion of the oligomerized stream through a hydrogenation step over a hydrogenation catalyst, for thereby producing said hydrocarbon stream comprising hydrocarbons boiling in the jet fuel range.

2. Process according to claim 1, wherein:
- step b) further comprises subsequently conducting a separation step, for thereby producing said oligomerized stream; and/or
- step c) further comprises subsequently conducting a separation step, for thereby producing said hydrocarbon stream comprising hydrocarbons boiling in the jet fuel range, optionally also comprising hydrocarbons boiling in the diesel fuel range.

3. Process according to any of claims 1-2, wherein in step a) in the MeOH synthesis catalyst, at least 80 wt%, e.g. at least 90 wt%, are oxides of Zn in combination with oxides of Zr and/or Al; and optionally, up to 20 wt% e.g. up to 10 wt% are oxides of any of: Si, Ti, Ce, La, Ga, In, Mo, Mn, Mg, Y.

4. Process according to any of claims 1-3, wherein in step a) the pressure is in the range 15-100 bar, such as 20-80 bar or 30-60 bar, and the temperature is in the range 240-400°C, such as 240-360°C, or 300-360°C.

5. Process according to any of claims 1-4, wherein the dual-function catalyst in step a) the ratio of MeOH synthesis catalyst to oxygenate conversion catalyst is between 10:1 and 1:10 by weight, such as between 5:1 and 1:5, such as between 2:1 and 1:2.

6. Process according to any of claims 1-5, wherein in step a) the MeOH synthesis catalyst and the oxygenate conversion catalyst are shaped as individual catalysts, and subsequently provided in the conversion reactor as a physical mixture.

7. Process according to any of claims 1-6, wherein prior to passing said syngas over said dual-function catalyst, the syngas passes over an upstream bed comprising a methanol synthesis catalyst; wherein the methanol synthesis catalyst is different from the MeOH synthesis catalyst of step a), or wherein the methanol synthesis catalyst is the same as the MeOH synthesis catalyst of step a).

8. Process according to claim 7, wherein the process further comprises directing a portion of the olefin product stream, suitably comprising light hydrocarbons such as ethane and/or ethylene, propane and/or propylene and butanes and/or butene, to step a), to a point upstream said dual-function catalyst.

9. Process according to claim 8, where said portion of the olefin product stream is directed to a point between said upstream bed comprising methanol synthesis catalyst and said fixed bed comprising a dual-function catalyst.

10. Process according to any of claims 7-9, wherein said portion of the olefin product stream is a first recycle stream comprising C2-C3 olefins or a C3 olefin stream which is withdrawn from said olefin product stream; and
wherein said first recycle stream is produced by the process further comprising, prior to step b):
- conducting the olefin product stream to a first separation step and withdrawing therefrom a liquid hydrocarbon fraction comprising at least 50 wt% of the C3-olefins contained in said olefin product stream, and withdrawing a gaseous fraction comprising C2-C3 olefins (C2=-C3=), suitably also comprising methane, ethane, propane, carbon monoxide, carbon dioxide and hydrogen, as said first recycle stream;
- conducting the liquid hydrocarbon fraction to a fractionation step and separating therefrom a C3 olefin product stream.

11. Process according to claim 10, wherein the first separation step further comprises withdrawing a water stream and the separation is conducted in a separation unit at 20-80°C, e.g. about 25°C, and 5-50 bar, such as 10-30 bar.

12. Process according to any of claims 10-11, wherein the fractionation step is a flash step being conducted in a flashing unit, such as a flash distillation unit, at 20-80°C, and 5-50 bar, such as 10-30 bar.

13. Process according to any of claims 2-12, wherein said oligomerization step b) comprises subsequently conducting said separation step; withdrawing therefrom a second recycle stream, said second recycle stream suitably being a stream rich in C8-olefins, suitably also comprising at least 50 wt% C8-olefins, and also comprising lower olefins than C8-olefins such as C5-C7 olefins, and n- and i-paraffins; and directing it to step a), such as to a point upstream said dual-functional catalyst, suitably between said upstream bed comprising methanol synthesis catalyst, and said fixed bed comprising a dual-function catalyst.

14. Process according to any of claims 1-13, wherein:
- the oligomerization catalyst in step b) is: a solid phosphoric acid ("SPA"), ion-exchange resins or a zeolite catalyst, for instance *MRE, BEA, FAU, MTT, TON, MFI and MTW catalyst, and the oligomerization step is conducted at a pressure of 30-100 bar, such as 50-100 bar, and a temperature of 100-350°C.

15. Process according to any of claims 1-14, wherein:
- the hydrogenation catalyst in step c) is:
a Ni-based hydrogenation catalyst, i.e. a hydrogenation catalyst containing Ni as the active metal, suitably a supported Ni catalyst having a Ni content of 1-25 wt% such as 10-15 wt%, based on the total weight of the catalyst, and wherein the support is selected from alumina, silica, titania and combinations thereof; or
a Cu-based hydrogenation catalyst, i.e. a hydrogenation catalyst containing Cu as the active metal, suitably a supported Cu-based catalyst having a Cu content of 10-75 wt%, suitably 12-38 wt% based on the total weight of the catalyst, and wherein the support is selected from alumina, zinc oxide, zinc aluminum spinel, silica, titania and combinations thereof;
and the hydrogenation step is suitably conducted at a pressure of 1-100 bar and a temperature of 0-350°C.

16. Process according to any of claims 1-15, wherein the oligomerization step (step b) and hydrogenation step (step c) are combined in a single hydro-oligomerization step (OLI/HYDRO), suitably wherein the OLI/HYDRO is conducted in a single reactor having a stacked reactor bed where a first bed comprises the oligomerization catalyst, e.g. zeolite catalyst, and a subsequent bed comprises the hydrogenation catalyst.

17. Process according to any of claims 1-16, further comprising, prior to step a):
- passing a water and/or steam stream through an electrolysis step for producing a hydrogen stream,
- providing a CO₂-rich stream, suitably containing at least 60 vol.% CO₂;
- optionally passing the CO₂-rich stream through an electrolysis step for producing a stream enriched in CO;
- combining said hydrogen stream with said CO₂-rich stream or with said stream enriched in CO, to form said synthesis gas; said synthesis gas suitably defining a CO₂/CO molar ratio of at least 2, preferably at least 5;
or
- passing a hydrocarbon feed stream such as a natural gas stream through a steam reforming step, suitably in an electrically heated steam methane reactor (e-SMR), for producing a raw synthesis gas, and optionally
- passing the raw synthesis gas through a secondary reforming unit under the addition of oxygen, for producing said synthesis gas; said synthesis gas suitably defining a CO₂/CO molar ratio of at least 2, preferably at least 5.

18. Plant for conducting the process according to any of claims 1-17, comprising:
- a fixed bed a conversion reactor arranged to receive a synthesis gas (syngas) stream comprising carbon dioxide and hydrogen, the fixed bed comprising a dual-function catalyst active in the conversion of syngas to the oxygenate(s) methanol (MeOH) and/or dimethyl ether (DME), and the conversion of said oxygenate(s) to an olefin product stream; wherein the dual-function catalyst comprises: a MeOH synthesis catalyst, in which the MeOH synthesis catalyst is a Cu and Cr-free catalyst comprising an oxide of Zn in combination with an oxide of any of Zr, Al, Si, Ti, Ce, La, Ga, In, Mo, Mn, Mg, Y, or combinations thereof; and an oxygenate conversion catalyst comprising a zeolite with a framework having a 10-ring pore structure, in which the 10-ring pore structure comprises a unidimensional (1-D) pore structure selected from any of: *MRE (ZSM-48), MTT (ZSM-23), TON (ZSM-22), or combinations thereof;
- an oligomerization reactor comprising an oligomerization catalyst, wherein the oligomerization reactor is arranged to receive at least a portion of the olefin product stream and withdraw an oligomerized stream;
- a hydrogenation reactor comprising a hydrogenation catalyst, wherein the hydrogenation reactor is arranged to receive at least a portion of the oligomerized stream and withdraw said hydrocarbon stream comprising hydrocarbons boiling in the jet fuel range.

## Patentansprüche

1. Prozess zur Herstellung eines Kohlenwasserstoffstroms, der Kohlenwasserstoffe mit einem Siedepunkt im Düsentreibstoffbereich umfasst, wobei der Prozess umfasst:
a) Leiten eines Synthesegas-, (Syngas)-Stroms, der Kohlendioxid und Wasserstoff umfasst, über ein Festbett in einem Umwandlungsreaktor, wobei das Festbett einen Doppelfunktionskatalysator umfasst, der bei der Umwandlung von Syngas in das/die Oxygenat(e) Methanol (MeOH) und/oder Dimethylether (DME) und der Umwandlung des/der Oxygenats/Oxygenate in einen Olefinproduktstrom aktiv ist; wobei der Doppelfunktionskatalysator umfasst: einen MeOH-Synthesekatalysator, wobei der MeOH-Synthesekatalysator ein Cu- und Cr-freier Katalysator ist, der ein Oxid von Zn in Kombination mit einem Oxid von einem von Zr, Al, Si, Ti, Ce, La, Ga, In, Mo, Mn, Mg, Y oder Kombinationen davon umfasst; und einen Oxygenatumwandlungskatalysator, der einen Zeolith mit einem Gerüst umfasst, das eine 10-Ring-Porenstruktur aufweist, wobei die 10-Ring-Porenstruktur eine eindimensionale (1-D) Porenstruktur umfasst, die ausgewählt ist aus: *MRE (ZSM-48), MTT (ZSM-23), TON (ZSM-22) oder Kombinationen davon;
b) Leiten mindestens eines Abschnitts des Olefinproduktstroms durch einen Oligomerisierungsschritt hindurch über einen Oligomerisierungskatalysator, um dadurch einen oligomerisierten Strom zu erzeugen;
c) Leiten mindestens eines Abschnitts des oligomerisierten Stroms durch einen Hydrierungsschritt hindurch über einen Hydrierungskatalysator, um dadurch den Kohlenwasserstoffstrom zu erzeugen, der Kohlenwasserstoffe umfasst, die im Düsentreibstoffbereich sieden.

2. Prozess nach Anspruch 1, wobei:
- Schritt b) weiter anschließend Durchführen eines Abscheideschritts umfasst, um dadurch den oligomerisierten Strom zu erzeugen; und/oder
- Schritt c) weiter anschließend Durchführen eines Abscheideschritts umfasst, um dadurch den Kohlenwasserstoffstrom zu erzeugen, der Kohlenwasserstoffe mit einem Siedepunkt im Düsentreibstoffbereich umfasst, optional auch Kohlenwasserstoffe mit einem Siedepunkt im Dieselkraftstoffbereich umfasst.

3. Prozess nach einem der Ansprüche 1-2, wobei in Schritt a) im MeOH-Synthesekatalysator mindestens 80 Gew.-%, z. B. mindestens 90 Gew.-% Oxide von Zn in Kombination mit Oxiden von Zr und/oder Al sind; und optional bis zu 20 Gew.-%, z. B. bis zu 10 Gew.-% Oxide von einem sind von: Si, Ti, Ce, La, Ga, In, Mo, Mn, Mg, Y.

4. Prozess nach einem der Ansprüche 1-3, wobei in Schritt a) der Druck im Bereich von 15-100 bar, beispielsweise 20-80 bar oder 30-60 bar, und die Temperatur im Bereich von 240-400 °C, beispielsweise 240-360 °C oder 300-360 °C liegt.

5. Prozess nach einem der Ansprüche 1-4, wobei der Doppelfunktionskatalysator in Schritt a) das Verhältnis von MeOH-Synthesekatalysator zu Oxygenat-Umwandlungskatalysator zwischen 10:1 und 1:10 nach Gewicht, beispielsweise zwischen 5:1 und 1:5, beispielsweise zwischen 2:1 und 1:2 ist.

6. Prozess nach einem der Ansprüche 1-5, wobei in Schritt a) der MeOH-Synthesekatalysator und der Oxygenat-Umwandlungskatalysator als individuelle Katalysatoren geformt und danach als physikalisches Gemisch im Umwandlungsreaktor bereitgestellt werden.

7. Prozess nach einem der Ansprüche 1-6, wobei das Syngas vor dem Leiten des Syngases über den Doppelfunktionskatalysator über ein stromaufwärts gelegenes Bett geleitet wird, das einen Methanolsynthesekatalysator umfasst; wobei sich der Methanolsynthesekatalysator von dem MeOH-Synthesekatalysator aus Schritt a) unterscheidet oder wobei der Methanolsynthesekatalysator derselbe ist wie der MeOH-Synthesekatalysator aus Schritt a).

8. Prozess nach Anspruch 7, wobei der Prozess weiter Lenken eines Abschnitts des Olefinproduktstroms umfasst, der geeigneterweise leichte Kohlenwasserstoffe wie Ethan und/oder Ethylen, Propan und/oder Propylen und Butane und/oder Buten umfasst, zu Schritt a) zu einem Punkt stromaufwärts des Doppelfunktionskatalysators.

9. Prozess nach Anspruch 8, wobei der Abschnitt des Olefinproduktstroms zu einem Punkt zwischen dem stromaufwärts gelegenen Bett, das einen Methanolsynthesekatalysator umfasst, und dem Festbett, das einen Doppelfunktionskatalysator umfasst, gelenkt wird.

10. Prozess nach einem der Ansprüche 7-9, wobei der Abschnitt des Olefinproduktstroms ein erster Rückführstrom ist, der C2-C3-Olefine umfasst, oder ein C3-Olefinstrom, der aus dem Olefinproduktstrom abgezogen wird; und
wobei der erste Rückführstrom durch den Prozess erzeugt wird, der vor Schritt b) weiter umfasst:
- Führen des Olefinproduktstroms zu einem ersten Abscheideschritt und Abziehen daraus einer flüssigen Kohlenwasserstofffraktion, die mindestens 50 Gew.-% der im Olefinproduktstrom enthaltenen C3-Olefine umfasst, und Abziehen einer gasförmigen Fraktion, die C2-C3-Olefine umfasst (C2=-C3=), geeigneterweise auch Methan, Ethan, Propan, Kohlenmonoxid, Kohlendioxid und Wasserstoff als erster Rückführstrom umfassen;
- Führen der flüssigen Kohlenwasserstofffraktion zu einem Fraktionierungsschritt und Abscheiden davon eines C3-Olefin-Produktstroms.

11. Prozess nach Anspruch 10, wobei der erste Abscheideschritt weiter Abziehen eines Wasserstroms umfasst und die Abscheidung in einer Abscheideeinheit bei 20-80 °C, beispielsweise etwa 25 °C, und 5-50 bar, beispielsweise 10-30 bar durchgeführt wird.

12. Prozess nach einem der Ansprüche 10-11, wobei der Fraktionierungsschritt ein Flash-Schritt ist, der in einer Flash-Einheit, beispielsweise einer Flash-Destillationseinheit, bei 20-80 °C und 5-50 bar, beispielsweise 10-30 bar durchgeführt wird.

13. Prozess nach einem der Ansprüche 2-12, wobei der Oligomerisierungsschritt b) anschließend Durchführen des Abscheideschritts umfasst; Abziehen eines zweiten Rückführstroms daraus, wobei der zweite Rückführstrom geeigneterweise ein an C8-Olefinen reicher Strom ist, geeigneterweise auch mindestens 50 Gew.-% C8-Olefine umfasst und auch niedrigere Olefine als C8-Olefine, beispielsweise C5-C7-Olefine, und n- und i-Paraffine umfasst; und Lenken desselben zu Schritt a), beispielsweise zu einem Punkt stromaufwärts des Doppelfunktionskatalysators, geeigneterweise zwischen dem stromaufwärts gelegenen Bett, das den Methanolsynthesekatalysator umfasst, und dem Festbett, das einen Doppelfunktionskatalysator umfasst.

14. Prozess nach einem der Ansprüche 1-13, wobei:
- der Oligomerisierungskatalysator in Schritt b) ist: eine feste Phosphorsäure ("SPA"), Ionenaustauscherharze oder ein Zeolithkatalysator, zum Beispiel *MRE, BEA-, FAU-, MTT-, TON-, MFI- und MTW-Katalysator, und der Oligomerisierungsschritt bei einem Druck von 30-100 bar, beispielsweise 50-100 bar, und einer Temperatur von 100-350 °C durchgeführt wird.

15. Prozess nach einem der Ansprüche 1-14, wobei:
- der Hydrierungskatalysator in Schritt c) ist:
ein Ni-basierter Hydrierungskatalysator, d. h. ein Hydrierungskatalysator, der Ni als aktives Metall enthält, geeigneterweise ein geträgerter Ni-Katalysator, der einen Ni-Gehalt von 1-25 Gew.-%, beispielsweise 10-15 Gew.-%, bezogen auf das Gesamtgewicht des Katalysators aufweist, und wobei der Träger aus Aluminiumoxid, Siliciumdioxid, Titandioxid und Kombinationen davon ausgewählt ist; oder
ein Cu-basierter Hydrierungskatalysator, d. h. ein Hydrierungskatalysator, der Cu als aktives Metall enthält, geeigneterweise ein geträgerter Cu-basierter Katalysator, der einen Cu-Gehalt von 10-75 Gew.-%, geeigneterweise 12-38 Gew.-%, basierend auf dem Gesamtgewicht des Katalysators aufweist, und wobei der Träger aus Aluminiumoxid, Zinkoxid, Zink-Aluminium-Spinell, Siliciumdioxid, Titandioxid und Kombinationen davon ausgewählt ist;
und der Hydrierungsschritt geeigneterweise bei einem Druck von 1-100 bar und einer Temperatur von 0-350 °C durchgeführt wird.

16. Prozess nach einem der Ansprüche 1-15, wobei der Oligomerisierungsschritt (Schritt b) und der Hydrierungsschritt (Schritt c) in einem einzigen Hydro-Oligomerisierungsschritt (OLI/HYDRO) kombiniert werden, wobei das OLI/HYDRO geeigneterweise in einem einzelnen Reaktor durchgeführt wird, der ein gestapeltes Reaktorbett aufweist, wobei ein erstes Bett den Oligomerisierungskatalysator, z. B. Zeolithkatalysator umfasst, und ein nachfolgendes Bett den Hydrierungskatalysator umfasst.

17. Prozess nach einem der Ansprüche 1-16, der weiter vor Schritt a) umfasst:
- Leiten von Wasser- und/oder Dampfstrom durch einen Elektrolyseschritt zum Erzeugen eines Wasserstoffstroms,
- Bereitstellen eines CO₂-reichen Stroms, der geeigneterweise mindestens 60 Vol.-% CO₂ enthält;
- optionales Leiten des CO₂-reichen Stroms durch einen Elektrolyseschritt zum Erzeugen eines mit CO angereicherten Stroms;
- Kombinieren des Wasserstoffstroms mit dem CO₂-reichen Strom oder mit dem mit CO angereicherten Strom, um das Synthesegas zu bilden; das Synthesegas geeigneterweise ein CO₂/CO-Molverhältnis von mindestens 2, vorzugsweise mindestens 5 definiert;
oder
- Leiten eines Kohlenwasserstoff-Zufuhrstroms, beispielsweise eines Erdgasstroms, durch einen Dampfreformierungsschritt, geeigneterweise in einem elektrisch beheizten Dampfmethanreaktor (e-SMR), zum Erzeugen eines Rohsynthesegases, und, optional,
- Leiten des Rohsynthesegases durch eine sekundäre Reformierungseinheit hindurch unter Zugabe von Sauerstoff, zum Erzeugen des Synthesegases; wobei das Synthesegas geeigneterweise ein CO₂/CO-Molverhältnis von mindestens 2, vorzugsweise mindestens 5 definiert.

18. Anlage zum Durchführen des Prozesses nach einem der Ansprüche 1-17, umfassend:
- einen Festbett-Umwandlungsreaktor, der angeordnet ist, um einen Synthesegas- (Syngas)-Strom aufzunehmen, der Kohlendioxid und Wasserstoff umfasst, wobei das Festbett einen Doppelfunktionskatalysator umfasst, der bei der Umwandlung von Syngas in das/die Oxygenat(e) Methanol (MeOH) und/oder Dimethylether (DME) und der Umwandlung des/der Oxygenats/Oxygenate in einen Olefinproduktstrom aktiv ist; wobei der Doppelfunktionskatalysator umfasst: einen MeOH-Synthesekatalysator, wobei der MeOH-Synthesekatalysator ein Cu- und Cr-freier Katalysator ist, der ein Oxid von Zn in Kombination mit einem Oxid von einem von Zr, Al, Si, Ti, Ce, La, Ga, In, Mo, Mn, Mg, Y oder Kombinationen davon umfasst; und einen Oxygenatumwandlungskatalysator, der einen Zeolith mit einem Gerüst umfasst, das eine 10-Ring-Porenstruktur aufweist, wobei die 10-Ring-Porenstruktur eine eindimensionale (1-D) Porenstruktur umfasst, die ausgewählt ist aus: *MRE (ZSM-48), MTT (ZSM-23), TON (ZSM-22) oder Kombinationen davon;
- einen Oligomerisierungsreaktor, der einen Oligomerisierungskatalysator umfasst, wobei der Oligomerisierungsreaktor angeordnet ist, um mindestens einen Abschnitt des Olefinproduktstroms aufzunehmen und einen oligomerisierten Strom abzuziehen;
- einen Hydrierungsreaktor, der einen Hydrierungskatalysator umfasst, wobei der Hydrierungsreaktor angeordnet ist, um mindestens einen Abschnitt des oligomerisierten Stroms aufzunehmen und den Kohlenwasserstoffstrom abzuziehen, der Kohlenwasserstoffe umfasst, die im Düsentreibstoffbereich sieden.

## Revendications

1. Procédé de production d'un flux d'hydrocarbures comprenant des hydrocarbures dont le point d'ébullition se situe dans la plage de carburéacteur, ledit procédé comprenant :
a) le fait de passer un flux de gaz de synthèse (syngaz) comprenant du dioxyde de carbone et de l'hydrogène sur un lit fixe dans un réacteur de conversion, le lit fixe comprenant un catalyseur à double fonction actif dans la conversion de syngaz en composé(s) oxygéné(s) de méthanol (MeOH) et/ou d'éther diméthylique (DME), et la conversion dudit ou desdits composés oxygénés en un flux de produit oléfinique ; dans lequel le catalyseur à double fonction comprend : un catalyseur de synthèse de MeOH, dans lequel le catalyseur de synthèse de MeOH est exempt de Cu et de Cr, comprenant un oxyde de Zn en combinaison avec un oxyde quelconque parmi Zr, AI, Si, Ti, Ce, La, Ga, In, Mo, Mn, Mg, Y, ou une combinaison de ceux-ci ; et un catalyseur de conversion de composé oxygéné comprenant une zéolite à structure poreuse à 10 cycles, dans lequel la structure poreuse à 10 cycles comprend une structure poreuse unidimensionnelle (1-D) choisie parmi : *MRE (ZSM-48), MTT (ZSM-23), TON (ZSM-22), ou des combinaisons de celles-ci ;
b) le fait de passer au moins une partie du flux de produit oléfinique à travers une étape d'oligomérisation au-dessus d'un catalyseur d'oligomérisation, pour produire ainsi un flux oligomérisé ;
c) le fait de passer au moins une partie du flux oligomérisé à travers une étape d'hydrogénation au-dessus du catalyseur d'hydrogénation, pour produire ainsi ledit flux d'hydrocarbures comprenant des hydrocarbures dont le point d'ébullition se situe dans la plage de carburéacteur.

2. Procédé selon la revendication 1, dans lequel :
- l'étape b) comprend en outre une réalisation ultérieure d'une étape de séparation, pour produire ainsi ledit flux oligomérisé ; et/ou
- l'étape c) comprend en outre une réalisation ultérieure d'une étape de séparation, pour produire ainsi ledit flux d'hydrocarbures comprenant des hydrocarbures dont le point d'ébullition se situe dans la plage de carburéacteur, comprenant également facultativement des hydrocarbures dont le point d'ébullition se situe dans la plage de carburant diesel.

3. Procédé selon l'une quelconque des revendications 1-2, dans lequel, à l'étape a), dans le catalyseur de synthèse de MeOH, au moins 80 % en poids, par exemple au moins 90 % en poids, sont des oxydes de Zn en combinaison avec des oxydes de Zr et/ou AI ; et facultativement, jusqu'à 20 % en poids, par exemple jusqu'à 10 % en poids, sont des oxydes d'un quelconque parmi : Si, Ti, Ce, La, Ga, In, Mo, Mn, Mg, Y.

4. Procédé selon l'une quelconque des revendications 1-3, dans lequel, à l'étape a), la pression est dans la plage de 15-100 bars, telle que 20-80 bars ou 30-60 bars, et la température est dans la plage de 240-400°C, telle que 240-360°C, ou 300-360°C.

5. Procédé selon l'une quelconque des revendications 1-4, dans lequel le catalyseur à double fonction, dans l'étape a), le rapport du catalyseur de synthèse de MeOH au catalyseur de conversion de composé oxygéné est compris entre 10:1 et 1:10 en poids, par exemple entre 5:1 et 1:5, comme entre 2:1 et 1:2.

6. Procédé selon l'une quelconque des revendications 1-5, dans lequel, dans l'étape a), le catalyseur de synthèse de MeOH et le catalyseur de conversion de composés oxygénés sont mis en forme sous forme de catalyseurs individuels, puis fournis dans le réacteur de conversion sous la forme d'un mélange physique.

7. Procédé selon l'une quelconque des revendications 1-6, dans lequel, avant de faire passer ledit syngaz au-dessus dudit catalyseur à double fonction, le syngaz passe au-dessus d'un lit en amont comprenant un catalyseur de synthèse de méthanol ; dans lequel le catalyseur de synthèse de méthanol est différent du catalyseur de synthèse de MeOH de l'étape a), ou dans lequel le catalyseur de synthèse de méthanol est le même que le catalyseur de synthèse de MeOH de l'étape a).

8. Procédé selon la revendication 7, dans lequel le procédé comprend en outre un acheminement d'une partie du flux de produit oléfinique, comprenant de manière appropriée des hydrocarbures légers tels que l'éthane et/ou l'éthylène, le propane et/ou le propylène et les butanes et/ou le butène, à l'étape a), jusqu'à un point en amont dudit catalyseur à double fonction.

9. Procédé selon la revendication 8, dans lequel ladite partie du flux de produit oléfinique est dirigée vers un point entre ledit lit en amont comprenant un catalyseur de synthèse de méthanol et ledit lit fixe comprenant un catalyseur à double fonction.

10. Procédé selon l'une quelconque des revendications 7-9, dans lequel ladite partie du flux de produit d'oléfine est un premier flux de recyclage comprenant des oléfines C2-C3 ou un flux d'oléfines C3 qui est retiré dudit flux de produit oléfinique ; et
dans lequel ledit premier flux de recyclage est produit par le procédé comprenant en outre, avant l'étape b) :
- la conduction du flux de produits oléfiniques vers une première étape de séparation et l'extraction de celui-ci d'une fraction d'hydrocarbure liquide comprenant au moins 50 % en poids des oléfines C3 contenues dans ledit flux de produit oléfinique, et l'extraction d'une fraction gazeuse comprenant des oléfines C2-C3 (C2=-C3=), comprenant également de manière appropriée du méthane, de l'éthane, du propane, du monoxyde de carbone, du dioxyde de carbone et de l'hydrogène, en tant que ledit premier flux de recyclage ;
- le conduction de la fraction d'hydrocarbure liquide vers une étape de fractionnement et la séparation de celle-ci d'un flux de produit oléfinique C3.

11. Procédé selon la revendication 10, dans lequel la première étape de séparation comprend en outre le retrait d'un flux d'eau, et la séparation est effectuée dans une unité de séparation à 20- 80°C, par exemple environ 25°C, et 5-50 bars, comme 10-30 bars.

12. Procédé selon l'une quelconque des revendications 10-11, dans lequel l'étape de fractionnement est une étape éclair réalisée dans une unité d'éclair, telle qu'une unité de distillation éclair, entre 20 et 80°C et 5 et 50 bars, comme 10-30 bars.

13. Procédé selon l'une quelconque des revendications 2-12, dans lequel ladite étape d'oligomérisation b) comprend une réalisation ultérieure de ladite étape de séparation ; un retrait à partir de celle-ci d'un second flux de recyclage, ledit second flux de recyclage étant de préférence un flux riche en oléfines C8, comprenant également de préférence au moins 50 % en poids d'oléfines C8, et comprenant également des oléfines inférieures aux oléfines C8 telles que des oléfines C5-C7, et des n- et i-paraffines ; et son acheminement vers l'étape a), par exemple vers un point en amont dudit catalyseur à double fonction, de préférence entre ledit lit en amont comprenant un catalyseur de synthèse de méthanol, et ledit lit fixe comprenant un catalyseur à double fonction.

14. Procédé selon l'une quelconque des revendications 1-13, dans lequel :
- le catalyseur d'oligomérisation à l'étape b) est : un acide phosphorique solide (« SPA »), des résines échangeuses d'ions ou un catalyseur zéolithique, par exemple un catalyseur *MRE, BEA, FAU, MTT, TON, MFI et MTW, et l'étape d'oligomérisation est réalisée à une pression de 30-100 bars, telle que 50-100 bars, et une température de 100-350°C.

15. Procédé selon l'une quelconque des revendications 1-14, dans lequel :
- le catalyseur d'hydrogénation à l'étape c) est :
un catalyseur d'hydrogénation à base de Ni, c'est-à-dire un catalyseur d'hydrogénation contenant du Ni comme métal actif, de préférence un catalyseur de Ni supporté présentant une teneur en Ni de 1-25 % en poids, par exemple de 10-15 % en poids, sur la base du poids total du catalyseur, et dans lequel le support est choisi parmi l'alumine, la silice, l'oxyde de titane et des combinaisons de ceux-ci ; ou
un catalyseur d'hydrogénation à base de Cu, c'est-à-dire un catalyseur d'hydrogénation contenant du Cu comme métal actif, de préférence un catalyseur à base de Cu supporté présentant une teneur en Cu de 10-75 % en poids, de manière appropriée de 12-38 % en poids sur la base du poids total du catalyseur, et dans lequel le support est choisi parmi l'alumine, l'oxyde de zinc, le spinelle de zinc et d'aluminium, la silice, l'oxyde de titane et des combinaisons de ceux-ci ;
et l'étape d'hydrogénation est réalisée de manière appropriée à une pression de 1-100 bars et à une température de 0-350°C.

16. Procédé selon l'une quelconque des revendications 1-15, dans lequel l'étape d'oligomérisation (étape b) et l'étape d'hydrogénation (étape c) sont combinées en une étape unique d'hydro-oligomérisation (OLI/HYDRO), de manière appropriée dans lequel la OLI/HYDRO est réalisée dans un réacteur unique présentant un lit de réacteur empilé dans lequel un premier lit comprend le catalyseur d'oligomérisation, par exemple un catalyseur à base de zéolite, et un lit suivant comprend le catalyseur d'hydrogénation.

17. Procédé selon l'une quelconque des revendications 1-16, comprenant en outre, avant l'étape a) :
- le fait de passer une eau et/ou un flux de vapeur à travers une étape d'électrolyse pour produire un flux d'hydrogène,
- la fourniture d'un flux riche en CO₂, contenant de manière appropriée au moins 60 % en volume de CO₂ ;
- facultativement, le fait de passer le flux riche en CO₂par une étape d'électrolyse pour produire un flux enrichi en CO ;
- la combinaison dudit flux d'hydrogène avec ledit flux riche en CO₂ou avec ledit flux enrichi en CO, pour former ledit gaz de synthèse ; ledit gaz de synthèse définissant de manière appropriée un rapport molaire de CO₂/CO d'au moins 2, de préférence d'au moins 5 ;
ou
- le fait de passer un flux d'alimentation d'hydrocarbures tel qu'un flux de gaz naturel à travers une étape de reformage à la vapeur, de manière appropriée dans un reformeur de méthane à la vapeur chauffé électriquement (e-SMR), pour produire un gaz de synthèse brut, et facultativement
- le fait de passer le gaz de synthèse brut à travers une unité de reformage secondaire sous ajout d'oxygène, pour produire ledit gaz de synthèse ; ledit gaz de synthèse définissant de manière appropriée un rapport molaire de COCO d'au moins 2, de préférence d'au moins 5.

18. Installation pour la mise en œuvre du procédé selon l'une quelconque des revendications 1-17, comprenant :
- un lit fixe dans un réacteur de conversion agencé pour recevoir un flux de gaz de synthèse (syngaz) comprenant du dioxyde de carbone et de l'hydrogène, le lit fixe comprenant un catalyseur à double fonction actif dans la conversion de syngaz en composé(s) oxygéné(s) de méthanol (MeOH) et/ou d'éther diméthylique (DME), et la conversion dudit ou desdits composés oxygénés en un flux de produit oléfinique ; dans lequel le catalyseur à double fonction comprend : un catalyseur de synthèse de MeOH, dans lequel le catalyseur de synthèse de MeOH est exempt de Cu et de Cr, comprenant un oxyde de Zn en combinaison avec un oxyde quelconque parmi Zr, AI, Si, Ti, Ce, La, Ga, In, Mo, Mn, Mg, Y, ou une combinaison de ceux-ci ; et un catalyseur de conversion de composé oxygéné comprenant une zéolite à structure poreuse à 10 cycles, dans lequel la structure poreuse à 10 cycles comprend une structure poreuse unidimensionnelle (1-D) choisie parmi : *MRE (ZSM-48), MTT (ZSM-23), TON (ZSM-22), ou des combinaisons de celles-ci ;
- un réacteur d'oligomérisation comprenant un catalyseur d'oligomérisation, dans lequel le réacteur d'oligomérisation est agencé pour recevoir au moins une partie du flux de produit oléfinique et retirer un flux oligomérisé ;
- un réacteur d'hydrogénation comprenant un catalyseur d'hydrogénation, dans lequel le réacteur d'hydrogénation est agencé pour recevoir au moins une partie du flux oligomérisé et retirer ledit flux d'hydrocarbures comprenant des hydrocarbures dont le point d'ébullition se situe dans la plage de carburéacteur.
